# EUROPEAN PATENT APPLICATION

(11) **EP 1 184 460 A1**
(43) Date of publication of application: **06.03.2002**
(21) Application number: 00307374.9
(22) Date of filing: 29.08.2000
(51) Int. Cl.: C12N 15/56, C12N 9/24, C12N 1/15, A23K 1/165

(54) **Modified fungal xylanases**

(71) Applicant: DSM N.V., 6411 TE Heerlen (NL)
(72) Inventor: Van den Hombergh, Johannes Petrus Theodorus W., 3454 AR De Meern (NL); Van der Laan, Ja Metske, 4839 AP Breda (NL); Menke, Hildegard Henna, 1103 TS Amsterdam z/o (NL); Daran, Jean-Marc Georges, 59800 Lille (FR)
(74) Representative: Wright, Simon Mark

(57) **Abstract**

Fungal xylanases are disclosed that have been modified to increase thermostability. The modifications are at exposed serine residues or within positions 90 to 160 (inclusive). The starting xylanase is the endo-1,4-β-xylanase I from *Aspergillus niger.* Single amino acid substitutions are preferred, in the B7, B8 or B9 anti-parallel strands of the β-sheet of the xylanase. Modifications can be at any of positions 91 to 95, 98, 103, 108 or 155, or at one or more of the serine residues 22, 27, 48, 49, 55, 59, 61, 173, 179 or 183, and the substitution can be a replacement of the original residue by a Cys, Thr, Asn, His, Arg or Asp residue. Complete DNA and amino acid sequences are disclosed for two of the mutants, S93L and S59N. The mutations can increase thermostability by more than ten-fold, and as the mutations are on the outside of the molecule, and away from the active site, they do not adversely affect the xylanase activity and the xylanases are still active under highly acidic conditions.

SEQUENCE LISTING

<110> DSM N.V.

<120> ENDOXYLANASE MUTANTS

<130> N78455 SMW

<140>
<141>

<160> 103

<170> PatentIn Ver. 2.1

<210> 1
<211> 636
<212> DNA
<213> Artificial Sequence

<220>
<221> CDS
<222> (1)..(636)

<220>
<223> Description of Artificial Sequence: mutated form
      of Aspergillus niger xylanase

<400> 1
atg aag gtc act gcg gct ttt gca ggt ctt ttg gtc acg gca ttc gcc    48
Met Lys Val Thr Ala Ala Phe Ala Gly Leu Leu Val Thr Ala Phe Ala
  1               5                  10                  15

gct cct gcc cca gaa cct gat ctg gtg tcg cga agt gcc ggt atc aac    96
Ala Pro Ala Pro Glu Pro Asp Leu Val Ser Arg Ser Ala Gly Ile Asn
             20                  25                  30

tac gtg caa aac tac aac ggc aac ctt ggt gat ttc acc tac gac gag    144
Tyr Val Gln Asn Tyr Asn Gly Asn Leu Gly Asp Phe Thr Tyr Asp Glu
         35                  40                  45

agt gcc gga aca ttt tcc atg tac tgg gaa gat gga gtg agc tcc gac    192
Ser Ala Gly Thr Phe Ser Met Tyr Trp Glu Asp Gly Val Ser Ser Asp
     50                  55                  60

ttt gtc gtt ggt ctg ggc tgg acc act ggt tct tct aac gct atc acc    240
Phe Val Val Gly Leu Gly Trp Thr Thr Gly Ser Ser Asn Ala Ile Thr
 65                  70                  75                  80

tac tct gcc gaa tac aac gct tct ggc tcc gct tcc tac ctc gct gtg    288
Tyr Ser Ala Glu Tyr Asn Ala Ser Gly Ser Ala Ser Tyr Leu Ala Val
                 85                  90                  95

tac ggc tgg gtc aac tat cct caa gct gag tac tac atc gtc gag gat    336
Tyr Gly Trp Val Asn Tyr Pro Gln Ala Glu Tyr Tyr Ile Val Glu Asp

100                 105                 110

tac ggt gat tat aac cct tgc agt tcg gcc aca agc ctt ggt acc gtg     384
Tyr Gly Asp Tyr Asn Pro Cys Ser Ser Ala Thr Ser Leu Gly Thr Val
        115                 120                 125

tac tct gat gga agc acc tac caa gtc tgc acc gac act cga aca aac     432
Tyr Ser Asp Gly Ser Thr Tyr Gln Val Cys Thr Asp Thr Arg Thr Asn
    130                 135                 140

gaa ccg tcc atc acg gga aca agc acg ttc acg cag tac ttc tcc gtt     480
Glu Pro Ser Ile Thr Gly Thr Ser Thr Phe Thr Gln Tyr Phe Ser Val
145                 150                 155                 160

cga gag agc acg cgc aca tct gga acg gtg act gtt gcc aac cat ttc     528
Arg Glu Ser Thr Arg Thr Ser Gly Thr Val Thr Val Ala Asn His Phe
                165                 170                 175

aac ttc tgg gcg cag cat ggg ttc ggc aat agc gac ttc aat tat cag     576
Asn Phe Trp Ala Gln His Gly Phe Gly Asn Ser Asp Phe Asn Tyr Gln
            180                 185                 190

gtc gtg gcg gtg gaa gca tgg agc ggt gct ggc agc gct agt gtc aca     624
Val Val Ala Val Glu Ala Trp Ser Gly Ala Gly Ser Ala Ser Val Thr
        195                 200                 205

atc tct tct tga                                                     636
Ile Ser Ser
    210

<210> 2
<211> 211
<212> PRT
<213> Artificial Sequence
<223> Description of Artificial Sequence: mutated form
      of Aspergillus niger xylanase

<400> 2
Met Lys Val Thr Ala Ala Phe Ala Gly Leu Leu Val Thr Ala Phe Ala
  1               5                  10                  15

Ala Pro Ala Pro Glu Pro Asp Leu Val Ser Arg Ser Ala Gly Ile Asn
             20                  25                  30

Tyr Val Gln Asn Tyr Asn Gly Asn Leu Gly Asp Phe Thr Tyr Asp Glu
         35                  40                  45

Ser Ala Gly Thr Phe Ser Met Tyr Trp Glu Asp Gly Val Ser Ser Asp
     50                  55                  60

Phe Val Val Gly Leu Gly Trp Thr Thr Gly Ser Ser Asn Ala Ile Thr
 65                  70                  75                  80

Tyr Ser Ala Glu Tyr Asn Ala Ser Gly Ser Ala Ser Tyr Leu Ala Val
                 85                  90                  95

Tyr Gly Trp Val Asn Tyr Pro Gln Ala Glu Tyr Tyr Ile Val Glu Asp

            100                 105                 110

Tyr Gly Asp Tyr Asn Pro Cys Ser Ser Ala Thr Ser Leu Gly Thr Val
        115                 120                 125

Tyr Ser Asp Gly Ser Thr Tyr Gln Val Cys Thr Asp Thr Arg Thr Asn
    130                 135                 140

Glu Pro Ser Ile Thr Gly Thr Ser Thr Phe Thr Gln Tyr Phe Ser Val
145                 150                 155                 160

Arg Glu Ser Thr Arg Thr Ser Gly Thr Val Thr Val Ala Asn His Phe
                165                 170                 175

Asn Phe Trp Ala Gln His Gly Phe Gly Asn Ser Asp Phe Asn Tyr Gln
            180                 185                 190

Val Val Ala Val Glu Ala Trp Ser Gly Ala Gly Ser Ala Ser Val Thr
        195                 200                 205

Ile Ser Ser
    210

<210> 3
<211> 685
<212> DNA
<213> Artificial Sequence

<220>
<223> Description of Artificial Sequence: mutated form
of Aspergillus niger xylanase

<400> 3
atgaaggtca ctgcggcttt tgcaggtctt ttggtcacgg cattcgccgc tcctgcccca 60
gaacctgatc tggtgtcgcg aagtgccggt atcaactacg tgcaaaacta caacggcaac 120
cttggtgatt tcacctacga cgagagtgcc ggaacatttt ccatgtactg ggaagatgga 180
gtgagctccg actttgtcgt tggtctgggc tggaccactg gttcttctaa gtgagtgact 240
gtattcttta accaaggtct aggatctaac gtctttcagc gctatcacct actctgccga 300
atacagcgct tctggctccg cttcctacct cgctgtgtac ggctgggtca actatcctca 360
agctgagtac tacatcgtcg aggattacgg tgattataac ccttgccttt cggccacaag 420
ccttggtacc gtgtactctg atggaagcac ctaccaagtc tgcaccgaca ctcgaacaaa 480
cgaaccgtcc atcacgggaa caagcacgtt cacgcagtac ttctccgttc gagagagcac 540
gcgcacatct ggaacggtga ctgttgccaa ccatttcaac ttctgggcgc agcatgggtt 600
cggcaatagc gacttcaatt atcaggtcgt ggcggtggaa gcatggagcg gtgctggcag 660
cgctagtgtc acaatctctt cttga 685

<210> 4
<211> 211
<212> PRT
<213> Artificial Sequence

<220>
<223> Description of Artificial Sequence: mutated form
of Aspergillus niger xylanase

<400> 4
Met Lys Val Thr Ala Ala Phe Ala Gly Leu Leu Val Thr Ala Phe Ala
1               5                   10                  15

Ala Pro Ala Pro Glu Pro Asp Leu Val Ser Arg Ser Ala Gly Ile Asn
            20                  25                  30

Tyr Val Gln Asn Tyr Asn Gly Asn Leu Gly Asp Phe Thr Tyr Asp Glu
        35                  40                  45

Ser Ala Gly Thr Phe Ser Met Tyr Trp Glu Asp Gly Val Ser Ser Asp
    50                  55                  60

Phe Val Val Gly Leu Gly Trp Thr Thr Gly Ser Ser Asn Ala Ile Thr
65                  70                  75                  80

Tyr Ser Ala Glu Tyr Ser Ala Ser Gly Ser Ala Ser Tyr Leu Ala Val
                85                  90                  95

Tyr Gly Trp Val Asn Tyr Pro Gln Ala Glu Tyr Tyr Ile Val Glu Asp
            100                 105                 110

Tyr Gly Asp Tyr Asn Pro Cys Leu Ser Ala Thr Ser Leu Gly Thr Val
        115                 120                 125

Tyr Ser Asp Gly Ser Thr Tyr Gln Val Cys Thr Asp Thr Arg Thr Asn
    130                 135                 140

Glu Pro Ser Ile Thr Gly Thr Ser Thr Phe Thr Gln Tyr Phe Ser Val
145                 150                 155                 160

Arg Glu Ser Thr Arg Thr Ser Gly Thr Val Thr Val Ala Asn His Phe
                165                 170                 175

Asn Phe Trp Ala Gln His Gly Phe Gly Asn Ser Asp Phe Asn Tyr Gln
            180                 185                 190

Val Val Ala Val Glu Ala Trp Ser Gly Ala Gly Ser Ala Ser Val Thr
        195                 200                 205

Ile Ser Ser
    210

<210> 5
<211> 36
<212> DNA
<213> Artificial Sequence

<220>
<223> Description of Artificial Sequence: Primer

<400> 5
ttcacacagg aaacacatat gagtgccggt atcaac                              36

<210> 6

<211> 39
<212> DNA
<213> Artificial Sequence

<220>
<223> Description of Artificial Sequence: Primer

<400> 6
cggcagagta ggtgatagcg ttagaagaac cagtggtcc                39


<210> 7
<211> 39
<212> DNA
<213> Artificial Sequence

<220>
<223> Description of Artificial Sequence: Primer

<400> 7
ggaccactgg ttcttctaac gctatcacct actctgccg                39


<210> 8
<211> 36
<212> DNA
<213> Artificial Sequence

<220>
<223> Description of Artificial Sequence: Primer

<400> 8
gacggccagt gccaagcttc aagaagagat tgtgac                36


<210> 9
<211> 36
<212> DNA
<213> Artificial Sequence

<220>
<223> Description of Artificial Sequence: Primer

<400> 9
ttcacacagg aaacacatat gaaggtcact gcggct                36


<210> 10
<211> 33
<212> DNA
<213> Artificial Sequence

<220>
<223> Description of Artificial Sequence: Primer

<400> 10
gatttgaaga agcgcgctag tgccggtatc aac                33

<210> 11
<211> 37
<212> DNA
<213> Artificial Sequence

<220>
<223> Description of Artificial Sequence: Primer

<400> 11
ctaagtataa attctcgagt caagaagaga ttgtgac                                37


<210> 12
<211> 33
<212> DNA
<213> Artificial Sequence

<220>
<223> Description of Artificial Sequence: Primer

<400> 12
gaagagacaa aagcttaaca cttgaaattt agg                                    33


<210> 13
<211> 34
<212> DNA
<213> Artificial Sequence

<220>
<223> Description of Artificial Sequence: Primer

<400> 13
agttgatacc ggcactagcg cgcttcttca aatc                                   34


<210> 14
<211> 21
<212> DNA
<213> Artificial Sequence

<220>
<223> Description of Artificial Sequence: Primer

<400> 14
gctgctttcg ctgacaagga t                                                 21


<210> 15
<211> 21
<212> DNA
<213> Artificial Sequence

<220>
<223> Description of Artificial Sequence: Primer

<400> 15

gataggaacg tcattatcgc a                                                21

<210> 16
<211> 33
<212> DNA
<213> Artificial Sequence

<220>
<223> Description of Artificial Sequence: Primer

<400> 16
tacatcgtcg aggattacgg tnnttataac cct                                    33

<210> 17
<211> 36
<212> DNA
<213> Artificial Sequence

<220>
<223> Description of Artificial Sequence: Primer

<400> 17
tacatcgtcg aggattacgg tgatnntaac ccttgc                                 36

<210> 18
<211> 39
<212> DNA
<213> Artificial Sequence

<220>
<223> Description of Artificial Sequence: Primer

<400> 18
tacatcgtcg aggattacgg tgattatnnc ccttgcagt                              39

<210> 19
<211> 21
<212> DNA
<213> Artificial Sequence

<220>
<223> Description of Artificial Sequence: Primer

<400> 19
accgtaatcc tcgacgatgt a                                                21

<210> 20
<211> 33
<212> DNA
<213> Artificial Sequence

<220>
<223> Description of Artificial Sequence: Primer

<400> 20
gaggattacg gtgattataa cnnttgcagt tcg                                    33

<210> 21
<211> 36
<212> DNA
<213> Artificial Sequence

<220>
<223> Description of Artificial Sequence: Primer

<400> 21
gaggattacg gtgattataa ccctnncagt tcggcc                                 36

<210> 22
<211> 39
<212> DNA
<213> Artificial Sequence

<220>
<223> Description of Artificial Sequence: Primer

<400> 22
gaggattacg gtgattataa cccttgcnnt tcggccaca                              39

<210> 23
<211> 21
<212> DNA
<213> Artificial Sequence

<220>
<223> Description of Artificial Sequence: Primer

<400> 23
gttataatca ccgtaatcct c                                                 21

<210> 24
<211> 33
<212> DNA
<213> Artificial Sequence

<220>
<223> Description of Artificial Sequence: Primer

<400> 24
ggtgattata acccttgcag tnnggccaca agc                                    33

<210> 25
<211> 36
<212> DNA
<213> Artificial Sequence

<220>
<223> Description of Artificial Sequence: Primer

<400> 25
ggtgattata acccttgcag ttcgnncaca agcctt                                36

<210> 26
<211> 39
<212> DNA
<213> Artificial Sequence

<220>
<223> Description of Artificial Sequence: Primer

<400> 26
ggtgattata acccttgcag ttcggccnna agccttggt                             39

<210> 27
<211> 21
<212> DNA
<213> Artificial Sequence

<220>
<223> Description of Artificial Sequence: Primer

<400> 27
actgcaaggg ttataatcac c                                                21

<210> 28
<211> 33
<212> DNA
<213> Artificial Sequence

<220>
<223> Description of Artificial Sequence: Primer

<400> 28
aacccttgca gttcggccac annccttggt acc                                   33

<210> 29
<211> 36
<212> DNA
<213> Artificial Sequence

<220>
<223> Description of Artificial Sequence: Primer

<400> 29
aacccttgca gttcggccac aagcnntggt accgtg                                36

<210> 30
<211> 39
<212> DNA

<213> Artificial Sequence

<220>
<223> Description of Artificial Sequence: Primer

<400> 30
aacccttgca gttcggccac aagccttnnt accgtgtac                    39


<210> 31
<211> 21
<212> DNA
<213> Artificial Sequence

<220>
<223> Description of Artificial Sequence: Primer

<400> 31
tgtggccgaa ctgcaagggt t                                       21


<210> 32
<211> 33
<212> DNA
<213> Artificial Sequence

<220>
<223> Description of Artificial Sequence: Primer

<400> 32
agttcggcca caagccttgg tnncgtgtac tct                          33


<210> 33
<211> 36
<212> DNA
<213> Artificial Sequence

<220>
<223> Description of Artificial Sequence: Primer

<400> 33
agttcggcca caagccttgg taccnngtac tctgat                       36


<210> 34
<211> 39
<212> DNA
<213> Artificial Sequence

<220>
<223> Description of Artificial Sequence: Primer

<400> 34
agttcggcca caagccttgg taccgtgnnc tctgatgga                    39


<210> 35

<211> 21
<212> DNA
<213> Artificial Sequence

<220>
<223> Description of Artificial Sequence: Primer

<400> 35
accaaggctt gtggccgaac t                                                21


<210> 36
<211> 33
<212> DNA
<213> Artificial Sequence

<220>
<223> Description of Artificial Sequence: Primer

<400> 36
acaagccttg gtaccgtgta cnntgatgga agc                                   33


<210> 37
<211> 36
<212> DNA
<213> Artificial Sequence

<220>
<223> Description of Artificial Sequence: Primer

<400> 37
acaagccttg gtaccgtgta ctctnntgga agcacc                                36


<210> 38
<211> 39
<212> DNA
<213> Artificial Sequence

<220>
<223> Description of Artificial Sequence: Primer

<400> 38
acaagccttg gtaccgtgta ctctgatnna agcacctac                             39


<210> 39
<211> 21
<212> DNA
<213> Artificial Sequence

<220>
<223> Description of Artificial Sequence: Primer

<400> 39
gtacacggta ccaaggcttg t                                                21

<210> 40
<211> 33
<212> DNA
<213> Artificial Sequence

<220>
<223> Description of Artificial Sequence: Primer

<400> 40
ggtaccgtgt actctgatgg anncacctac caa                                    33


<210> 41
<211> 36
<212> DNA
<213> Artificial Sequence

<220>
<223> Description of Artificial Sequence: Primer

<400> 41
ggtaccgtgt actctgatgg aagcnnctac caagtc                                 36


<210> 42
<211> 39
<212> DNA
<213> Artificial Sequence

<220>
<223> Description of Artificial Sequence: Primer

<400> 42
ggtaccgtgt actctgatgg aagcaccnnc caagtctgc                              39


<210> 43
<211> 21
<212> DNA
<213> Artificial Sequence

<220>
<223> Description of Artificial Sequence: Primer

<400> 43
tccatcagag tacacggtac c                                                 21


<210> 44
<211> 33
<212> DNA
<213> Artificial Sequence

<220>
<223> Description of Artificial Sequence: Primer

<400> 44

tactctgatg gaagcaccta cnnagtctgc acc 33

<210> 45
<211> 36
<212> DNA
<213> Artificial Sequence

<220>
<223> Description of Artificial Sequence: Primer

<400> 45
tactctgatg gaagcaccta ccaannctgc accgac 36

<210> 46
<211> 39
<212> DNA
<213> Artificial Sequence

<220>
<223> Description of Artificial Sequence: Primer

<400> 46
tactctgatg gaagcaccta ccaagtcnnc accgacact 39

<210> 47
<211> 21
<212> DNA
<213> Artificial Sequence

<220>
<223> Description of Artificial Sequence: Primer

<400> 47
gtaggtgctt ccatcagagt a 21

<210> 48
<211> 33
<212> DNA
<213> Artificial Sequence

<220>
<223> Description of Artificial Sequence: Primer

<400> 48
ggaagcacct accaagtctg cnncgacact cga 33

<210> 49
<211> 36
<212> DNA
<213> Artificial Sequence

<220>
<223> Description of Artificial Sequence: Primer

<400> 49
ggaagcacct accaagtctg caccnncact cgaaca                                36

<210> 50
<211> 39
<212> DNA
<213> Artificial Sequence

<220>
<223> Description of Artificial Sequence: Primer

<400> 50
ggaagcacct accaagtctg caccgacnnt cgaacaaac                             39

<210> 51
<211> 21
<212> DNA
<213> Artificial Sequence

<220>
<223> Description of Artificial Sequence: Primer

<400> 51
gcagacttgg taggtgcttc c                                                21

<210> 52
<211> 33
<212> DNA
<213> Artificial Sequence

<220>
<223> Description of Artificial Sequence: Primer

<400> 52
taccaagtct gcaccgacac tnnaacaaac gaa                                   33

<210> 53
<211> 36
<212> DNA
<213> Artificial Sequence

<220>
<223> Description of Artificial Sequence: Primer

<400> 53
taccaagtct gcaccgacac tcgannaaac gaaccg                                36

<210> 54
<211> 39
<212> DNA
<213> Artificial Sequence

<220>
<223> Description of Artificial Sequence: Primer

<400> 54
taccaagtct gcaccgacac tcgaacannc gaaccgtcc                                39


<210> 55
<211> 21
<212> DNA
<213> Artificial Sequence

<220>
<223> Description of Artificial Sequence: Primer

<400> 55
agtgtcggtg cagacttggt a                                                   21


<210> 56
<211> 33
<212> DNA
<213> Artificial Sequence

<220>
<223> Description of Artificial Sequence: Primer

<400> 56
tgcaccgaca ctcgaacaaa cnnaccgtcc atc                                      33


<210> 57
<211> 36
<212> DNA
<213> Artificial Sequence

<220>
<223> Description of Artificial Sequence: Primer

<400> 57
tgcaccgaca ctcgaacaaa cgaanngtcc atcacg                                   36


<210> 58
<211> 39
<212> DNA
<213> Artificial Sequence

<220>
<223> Description of Artificial Sequence: Primer

<400> 58
tgcaccgaca ctcgaacaaa cgaaccgnnc atcacggga                                39


<210> 59
<211> 21
<212> DNA

<213> Artificial Sequence

<220>
<223> Description of Artificial Sequence: Primer

<400> 59
gtttgttcga gtgtcggtgc a                                                21


<210> 60
<211> 33
<212> DNA
<213> Artificial Sequence

<220>
<223> Description of Artificial Sequence: Primer

<400> 60
actcgaacaa acgaaccgtc cnncacggga aca                                   33


<210> 61
<211> 36
<212> DNA
<213> Artificial Sequence

<220>
<223> Description of Artificial Sequence: Primer

<400> 61
actcgaacaa acgaaccgtc catcnnggga acaagc                                36


<210> 62
<211> 39
<212> DNA
<213> Artificial Sequence

<220>
<223> Description of Artificial Sequence: Primer

<400> 62
actcgaacaa acgaaccgtc catcacgnna acaagcacg                             39


<210> 63
<211> 21
<212> DNA
<213> Artificial Sequence

<220>
<223> Description of Artificial Sequence: Primer

<400> 63
ggacggttcg tttgttcgag t                                                21


<210> 64

<211> 33
<212> DNA
<213> Artificial Sequence

<220>
<223> Description of Artificial Sequence: Primer

<400> 64
aacgaaccgt ccatcacggg annaagcacg ttc                                33


<210> 65
<211> 36
<212> DNA
<213> Artificial Sequence

<220>
<223> Description of Artificial Sequence: Primer

<400> 65
aacgaaccgt ccatcacggg aacanncacg ttcacg                             36


<210> 66
<211> 39
<212> DNA
<213> Artificial Sequence

<220>
<223> Description of Artificial Sequence: Primer

<400> 66
aacgaaccgt ccatcacggg aacaagcnng ttcacgcag                          39


<210> 67
<211> 21
<212> DNA
<213> Artificial Sequence

<220>
<223> Description of Artificial Sequence: Primer

<400> 67
tcccgtgatg gacggttcgt t                                             21


<210> 68
<211> 33
<212> DNA
<213> Artificial Sequence

<220>
<223> Description of Artificial Sequence: Primer

<400> 68
acgcagtact tctccgttcg anngagcacg cgc                                33

<210> 69
<211> 36
<212> DNA
<213> Artificial Sequence

<220>
<223> Description of Artificial Sequence: Primer

<400> 69
acgcagtact tctccgttcg agagnncacg cgcaca                                36


<210> 70
<211> 39
<212> DNA
<213> Artificial Sequence

<220>
<223> Description of Artificial Sequence: Primer

<400> 70
acgcagtact tctccgttcg agagagcnng cgcacatct                             39


<210> 71
<211> 21
<212> DNA
<213> Artificial Sequence

<220>
<223> Description of Artificial Sequence: Primer

<400> 71
tcgaacggag aagtactgcg t                                                21


<210> 72
<211> 33
<212> DNA
<213> Artificial Sequence

<220>
<223> Description of Artificial Sequence: Primer

<400> 72
ttctccgttc gagagagcac gnncacatct gga                                   33


<210> 73
<211> 36
<212> DNA
<213> Artificial Sequence

<220>
<223> Description of Artificial Sequence: Primer

<400> 73

ttctccgttc gagagagcac gcgcnnatct ggaacg                                36

<210> 74
<211> 39
<212> DNA
<213> Artificial Sequence

<220>
<223> Description of Artificial Sequence: Primer

<400> 74
ttctccgttc gagagagcac gcgcacannt ggaacggtg                             39

<210> 75
<211> 21
<212> DNA
<213> Artificial Sequence

<220>
<223> Description of Artificial Sequence: Primer

<400> 75
cgtgctctct cgaacggaga a                                                21

<210> 76
<211> 33
<212> DNA
<213> Artificial Sequence

<220>
<223> Description of Artificial Sequence: Primer

<400> 76
cgagagagca cgcgcacatc tnnaacggtg act                                   33

<210> 77
<211> 36
<212> DNA
<213> Artificial Sequence

<220>
<223> Description of Artificial Sequence: Primer

<400> 77
cgagagagca cgcgcacatc tggannggtg actgtt                                36

<210> 78
<211> 39
<212> DNA
<213> Artificial Sequence

<220>
<223> Description of Artificial Sequence: Primer

<400> 78
cgagagagca cgcgcacatc tggaacgnng actgttgcc                                39

<210> 79
<211> 21
<212> DNA
<213> Artificial Sequence

<220>
<223> Description of Artificial Sequence: Primer

<400> 79
agatgtgcgc gtgctctctc g                                                   21

<210> 80
<211> 33
<212> DNA
<213> Artificial Sequence

<220>
<223> Description of Artificial Sequence: Primer

<400> 80
acgcgcacat ctggaacggt gnntgttgcc aac                                      33

<210> 81
<211> 36
<212> DNA
<213> Artificial Sequence

<220>
<223> Description of Artificial Sequence: Primer

<400> 81
acgcgcacat ctggaacggt gactnntgcc aaccat                                   36

<210> 82
<211> 39
<212> DNA
<213> Artificial Sequence

<220>
<223> Description of Artificial Sequence: Primer

<400> 82
acgcgcacat ctggaacggt gactgttnnc aaccatttc                                39

<210> 83
<211> 21
<212> DNA
<213> Artificial Sequence

<220>
<223> Description of Artificial Sequence: Primer

<400> 83
caccgttcca gatgtgcgcg t                                                21


<210> 84
<211> 33
<212> DNA
<213> Artificial Sequence

<220>
<223> Description of Artificial Sequence: Primer

<400> 84
tctggaacgg tgactgttgc cnnccatttc aac                                   33


<210> 85
<211> 36
<212> DNA
<213> Artificial Sequence

<220>
<223> Description of Artificial Sequence: Primer

<400> 85
tctggaacgg tgactgttgc caacnntttc aacttc                                36


<210> 86
<211> 39
<212> DNA
<213> Artificial Sequence

<220>
<223> Description of Artificial Sequence: Primer

<400> 86
tctggaacgg tgactgttgc caaccatnnc aacttctgg                             39


<210> 87
<211> 21
<212> DNA
<213> Artificial Sequence

<220>
<223> Description of Artificial Sequence: Primer

<400> 87
ggcaacagtc accgttccag a                                                21


<210> 88
<211> 33
<212> DNA

<213> Artificial Sequence

<220>
<223> Description of Artificial Sequence: Primer

<400> 88
gtgactgttg ccaaccattt cnncttctgg gcg                                33


<210> 89
<211> 36
<212> DNA
<213> Artificial Sequence

<220>
<223> Description of Artificial Sequence: Primer

<400> 89
gtgactgttg ccaaccattt caacnnctgg gcgcag                             36


<210> 90
<211> 39
<212> DNA
<213> Artificial Sequence

<220>
<223> Description of Artificial Sequence: Primer

<400> 90
gtgactgttg ccaaccattt caacttcnng gcgcagcat                          39


<210> 91
<211> 21
<212> DNA
<213> Artificial Sequence

<220>
<223> Description of Artificial Sequence: Primer

<400> 91
gaaatggttg gcaacagtca c                                             21


<210> 92
<211> 33
<212> DNA
<213> Artificial Sequence

<220>
<223> Description of Artificial Sequence: Primer

<400> 92
gccaaccatt tcaacttctg gnngcagcat ggg                                33


<210> 93

<211> 36
<212> DNA
<213> Artificial Sequence

<220>
<223> Description of Artificial Sequence: Primer

<400> 93
gccaaccatt tcaacttctg ggcgnngcat gggttc                                36


<210> 94
<211> 39
<212> DNA
<213> Artificial Sequence

<220>
<223> Description of Artificial Sequence: Primer

<400> 94
gccaaccatt tcaacttctg ggcgcagnnt gggttcggc                             39


<210> 95
<211> 21
<212> DNA
<213> Artificial Sequence

<220>
<223> Description of Artificial Sequence: Primer

<400> 95
ccagaagttg aaatggttgg c                                                21


<210> 96
<211> 33
<212> DNA
<213> Artificial Sequence

<220>
<223> Description of Artificial Sequence: Primer

<400> 96
ttcaacttct gggcgcagca tnngttcggc aat                                   33


<210> 97
<211> 36
<212> DNA
<213> Artificial Sequence

<220>
<223> Description of Artificial Sequence: Primer

<400> 97
ttcaacttct gggcgcagca tgggnncggc aatagc                                36

<210> 98
<211> 39
<212> DNA
<213> Artificial Sequence

<220>
<223> Description of Artificial Sequence: Primer

<400> 98
ttcaacttct gggcgcagca tgggttcnnc aatagcgac                                39


<210> 99
<211> 21
<212> DNA
<213> Artificial Sequence

<220>
<223> Description of Artificial Sequence: Primer

<400> 99
atgctgcgcc cagaagttga a                                                   21


<210> 100
<211> 33
<212> DNA
<213> Artificial Sequence

<220>
<223> Description of Artificial Sequence: Primer

<400> 100
tgggcgcagc atgggttcgg cnntagcgac ttc                                      33


<210> 101
<211> 36
<212> DNA
<213> Artificial Sequence

<220>
<223> Description of Artificial Sequence: Primer

<400> 101
tgggcgcagc atgggttcgg caatnncgac ttcaat                                   36


<210> 102
<211> 39
<212> DNA
<213> Artificial Sequence

<220>
<223> Description of Artificial Sequence: Primer

<400> 102

tgggcgcagc atgggttcgg caatagcnnc ttcaattat 39

<210> 103
<211> 21
<212> DNA
<213> Artificial Sequence

<220>
<223> Description of Artificial Sequence: Primer

<400> 103
gccgaaccca tgctgcgccc a 21

## Description

### Field of Invention

The present invention relates to a modified xylanases, such as those based on *Aspergillus* xylanases and their use in degrading xylan in cellulose. The xylanases find use in baking, in animal feed (to improve feed conversion) and in paper production.

### Background of the invention

The composition of a plant cell wall is complex and variable and contains several carbohydrate biopolymers. Polysaccharides are mainly found in the form of long chains of cellulose (the main structural component of the plant cell wall), hemicellulose (comprising various β-xylan chains, such as xyloglucans) pectin and lignin. The most abundant hemicelluloses are xylans and their derivatives such as arabinoxylan and xyloglycan.

Plant hemicelluloses include xylan, arabinoxylan, glucuronoarabinoxylan and xyloglucan. Xylan (CAS Registry No. 9014-63-5) consists of a backbone of β-1,4-linked D-xylopyranosyl units, optionally substituted with side chains such as arabinose and/or glucuronic acid residues. The structure is:
→4)-β-D-Xyl*p*-(1→4)-β-D-Xyl*p*(2←1A)-(1→4)-β-D-Xyl*p*-(1→4)-β-D-Xyl*p*(3←1B)-(1→

(Xyl*p* = xylopyranosyl unit; A = α-(4-*O*)-methyl-(D-glucuronopyranosyl) unit, sometime an acetyl; and B = a-(L-arabinofuranosyl) unit, sometimes an acetyl).

Xylans may represent more than 30% of the dry weight of terrestrial plants. Hence xylan is an important component of materials from natural sources that are used in industrial processes ranging from baking, improvement of animal feed conversion and paper production.

Several enzymes can be used to hydrolyse xylan biopolymers. The most important are endo-1,4-β-xylanases (EC 3.2.1.8). These xylanases can degrade a xylan to short xylo-oligosaccharides of varying lengths. Enzymatic degradation of these polymers into shorter fragments increases solubility, reduces viscosity and can liberate water that is bound to the polymers.

Microbial xylanases are of interest in the food and feed industry, particularly in bio-bleaching. Xylanases have been isolated from fungi bacteria and yeast. Fungal xylanases have a pH optimum in the range of pH 2 to 6, whereas bacterial xylanases have pH optimum in the range 5 to 8, with some extremes of up to pH 10. Thus bacterial xylanases are often less acid stable than their fungal counterparts, but in compensation they are effective at higher temperatures. Despite this bacterial xylanases have not replaced their fungal counterparts in industrial applications because these often occur at a pH lower than that at which bacterial xylanases can function efficiently. Thus fungal xylanases are ideally suited for industrial processes conducted at low pH.

Endo-1,4-β-xylanases (EC 3.2.1.8) have been classified into two families based upon their amino acid sequence and three dimensional structure. These are Family 10 (formerly F-type) and Family 11 (formerly G-type). The latter family is of most interest for animal feed, and xylanases from bacteria, yeast or fungi in this family all have a very similar three dimensional structure.

Generally, Family 11 xylanases have a single catalytic domain of about 21kD, which comprises mainly β strands that are arranged in two anti-parallel β-sheets. The larger sheet is highly twisted and forms a long cleft which can accommodate the xylan polymer. The only alpha helix that is present is imbedded on the rear side on this sheet. In some cases larger Family 11 xylanases with additional non-catalytic domains have been reported.

The G-type xylanases can be further subdivided according to pH optima, which can be correlated to the residue that is adjacent to the acid/base catalyst at the active site. This residue is aspartic acid in xylanases that function optimally under acidic conditions.

A xylanase from *Aspergillus kawachii* has been mutated by single substitution of aspartic acid to asparagine and this elevated its pH optimum from 2 to 5¹. The xylanase C was mutated four times (B37N, S, E and K¹). In the asparagine mutant the optimum pH shifted to alkaline (pH 5.0) and the activity decreased by 15% compared to the wild-type. In a *Bacillus circulans* xylanase the reverse substitution of asparagine to aspartic acid shifted the pH optimum from 5.7 to 4.6². Thus within Family 11 there are acidic (or aspartic acid) modulated xylanases and moderately acid to alkaline (or asparagine) modulated xylanases.

Family 11 xylanases have been mutagenised. Apart from exploring the catalytic mechanism and pH dependence, the effect on thermostability has also been investigated. Thermostable mutants have been reported for *Bacillus pumilus* and *Bacillus circulans*, and both of these are bacterial xylanases which are moderately acidic to neutral (and asparagine modulated). Thermostable mutants have also been suggested for *Trichoderma reesei,* and although this is a fungus, it exhibits the asparagine modulated slight acidic to neutral pH optimum.

Chemical mutagens have been used to produce four heat resistant mutants of xylanase A from *B. pumilus*³. The four mutants were N56 (S26W, G38D and T126S), N102 (G38D), N104 (G38S and R48K) and F1 (S12K). These are bacterial rather than fungal xylanases and have a neutral pH optimum of about 6.5. Position 38 corresponds to Y29 in the endoxylanase I enzyme from *A. niger* on which the modified proteins of the invention are based (and so this means that S12 corresponds to G3, R48 corresponds to G41, S26 corresponds to D16 and T126 corresponds to D113).

(Note that in this specification mutations are indicated by using the single letter code for the amino acid residues. The original amino acid (present in the wild-type) is indicated first, followed by the numerical position in the (usually mature) amino acid sequence, and then a single letter indicating the new amino acid at that position that has been used to substitute the original).

Site-specific mutagenesis of Family 11 from *B. circulans* has been used to alter thermomostability. New disulphide bonds at 98 to 152 and 100 to 148 improved resistance to thermoinactivation⁴. A disulphide bond has been introduced by adding cysteine residues at the N-terminus and extra residues G187 and G188 at the C-terminus⁵. The positions S100 and N148 correspond to S103 and N147 respectively in the *A. niger* endoxylanase I. While in theory the introduction of a disulphide bridge at these corresponding locations might have suggested an increase in thermostability, when such a mutant was prepared (substitutions S103C and N147C) no improved thermostability was in fact found (Comparative Example 2). For this reason the introduction of a disulphide bridge (such as at S103→N147) is not considered to be a modification that increases thermostability. Presumably several structural differences between the two enzymes account for a contribution to thermostability in *B. circulans* while a slight destabilisation was observed for the *A. niger* endoxylanase. This illustrates that (thermo) stabilising mutations cannot always be transferred from one species of xylanase to another, unless there are factors such as if the local environment at or around a substitution is highly conserved.

Three additional mutations near the N-terminus of the *B. circulans* xylanase namely T3G, D4Y/F and M8Y/F improved thermostability⁴ (these correspond to positions I4, N5 and N9 in *A. niger* endoxylanase). A further S22P mutation has also been mentioned⁶. Although alignment suggests that position 22 corresponds with glutamic acid at position 21, the 3D structure show that due to an insertion at position 22 in the *A. niger* endoxylanase, in fact there is no corresponding position for S22 of the *B. circulans* enzyme in both fungal xylanases. These four substitutions again emphasise the importance of the context in which the substitutions are made, for example xylanase XynII of *T. reesei* possesses all four of these residues and yet it is only mediocre in thermostability⁷.

The thermostability of this T. *reesei* enzyme⁸ in mutants N38E, T and Y is of interest since the corresponding position in *A. niger* xylanase (Y29) already contains the tyrosine residue. Other mutations (N10H, Y27M and M27L) increased thermostability⁷. Extension of the N-terminus or replacement of the N-terminal 20 to 30 amino acids by the corresponding sequence from *Thermonospora fusca* improved thermostability, but also exhibited a shift in pH optimum to alkali conditions, which is disadvantageous when performance under acidic conditions is required by industry.

There is therefore a need for a xylanase that not only has increased thermostability (over the wild-type), but is also active at low pH. The invention therfore seeks to provide modified xylanases with better thermostability but without an undesirable shift of the optimum pH to neutral or alkaline conditions.

### Summary of the Invention

Novel proteins comprising a modified xylanases are now provided which are able to cleave xylan such as present in plant material. These are up to 10 times or more thermostable than their unmodified counterparts, and yet retain a pH optimum that is acidic. The present invention also provides a polynucleotide which encodes the proteins.

The invention also provides:
- an (eg. expression) vector which comprises a polynucleotide of the invention and which may be capable of expressing a protein of the invention;
- a cell line comprising a vector of the invention;
- a method of producing a protein of the invention which method comprises maintaining a cell line of the invention under conditions suitable for obtaining expression of the protein and, if necessary, isolating the protein; and
- a method of degrading xylan, the method comprising contacting a material comprising xylan with a protein of the invention.

### Brief Description of the Sequences

SEQ ID No. 1 is a DNA sequence encoding a first protein of the invention (modified *A. niger* endoxylanase I with a single mutation, S59N, and underneath the corresponding amino acid sequence);
SEQ ID No. 2 is the amino acid sequence of the first protein;
SEQ ID No. 3 is a DNA sequence with an intron from 231 to 279 encoding a second protein of the invention (another modified xylanase, this time with the mutation S93L);
SEQ ID No. 4 is the amino acid sequence of the second protein; and
SEQ ID Nos. 5 to 103 are oligonucleotides that were used to modify the xylanases.

### Brief description of the Figures

Figure 1 is an alignment of six prior art xylanase amino acid sequences¹¹, including the sequence of the *A. niger* xylanase (known in the art as EndoI) from which the modified xylanases of the invention are derived;
Figure 2 is a diagram of the overall structure of a *T. lagunosis* xylanase⁹ which has a structure similar to the xylanase from *A. niger;*

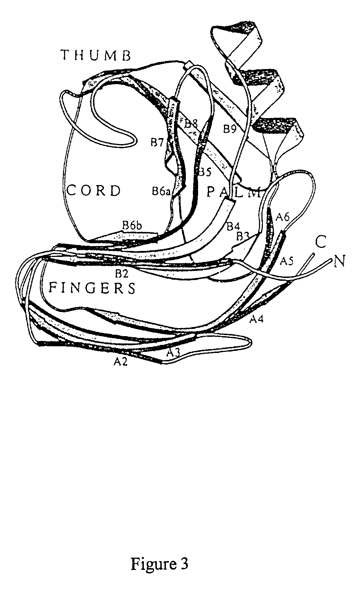

Figure 3 is a topology diagram¹⁰ of the XYNI xylanase from *T. reeseii;*
Figure 4 is a schematic representation of the intron free xlnA gene by PCR;
Figure 5 is an alignment often prior art xylanase sequences, the first (UKR_A) being that of the *A. niger* xylanase; and
Figure 6 is a topology cartoon of the *A. niger* xylanase.

### Detailed Description of the Invention

### A. Proteins

A first aspect of the present invention relates to a protein comprising a modified xylanase. Preferably the xylanase that is modified is a fungal xylanase, that is to say it is of fungal origin or derived from a fungal species. The modification will be to increase thermostability when compared with the unmodified xylanase, in other words the naturally occuring enzyme (usually called the wild-type). The (or each) modification can be at an exposed serine residue or within residues 90 to 160.

The invention also relates to a xylanase that is at least twice as thermostable as an *A. niger* xylanase and has a pH optimum in the range of from pH 2 to 4.

The modification may be an insertion, deletion or substitution, but is preferably the latter. The number of modifications may be 10 or less, such as 5 or less, such as 3 or less. However, optimally there are either 1 or 2 modifications (when compared to the wild-type). Although the modification can increase thermostability, this preferably does not come at the expense of activity, and preferably the modified xylanase is no less active then the unmodified xylanase. This can be achieved by making "non-interfering" modifications, that only improve the properties of the xylanase, rather than decrease a property such as activity. Thus modifications are preferred that do not alter (substantially or significantly) the structure of the xylanase. This can therefore exclude major modifications such as the introduction of a new disulphide bridge.

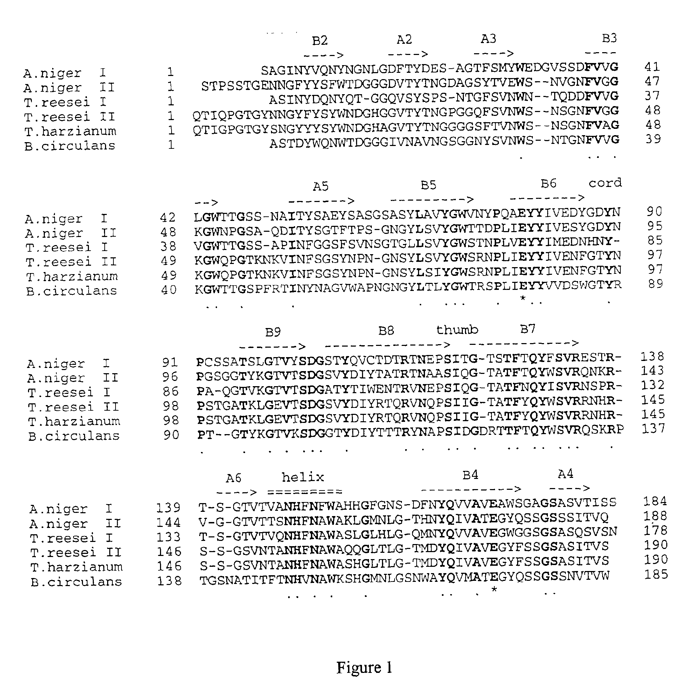

The modification(s) may be within residues 90 to 160, inclusive. This numbering is based on the wild-type enzyme. The full sequence of six different xylanases, including one of the xylanases on which the modified proteins of the invention is based, is shown in Figure 1. The invention however is not limited to the modification of one specific enzyme, and therefore while the numbering is based on the endo-1,4-β-xylanase from *A. niger,* the same modification in a different xylanase at a corresponding position is within the scope of the invention.

Serine residues that are exposed are usually ones that are at or (very) near the outside of the xylanase. This means that they may protrude from the surface of the enyzme, such as into the surrounding environment. They may be contactable by solvent, and indeed solvent accessability is one measure of how exposed a serine residue is. This variable is detailed in the Examples later: preferably the solvent exposure (Acc) is at least zero, preferably at least 20, such as at least 70, optimally at least 100.

Preferably the xylanase is modified so that thermostability is at least 1.5 times as much, such as at least twice as much, as the unmodified enzyme. However, modified xylanases have been made that have even larger increases in the thermostability, and these may be up to at least 3 times, at least 4 times, at least 5 times or optimally at least 6 times as thermostable as the unmodified enzyme.

The modified xylanases of the invention are based on fungal xylanases. This is in contrast to some workers in the field who have modified bacterial xylanases, such as from *Bacillus.* The xylanase is preferably from a fungal species from *Aspergillus*. In particular, the xylanase is from *Asp. niger* or *Asp. tubingensis*. Preferably the xylanase is an endoxylanase. Optimally it is an endo-1,4-β-xylanase. A preferred xylanase for modification is therefore the endo-1,4-β-xylanase I (*xlnA* I enzyme) such as from *A. niger,* suitably strain DS16813, for example the strain deposited under the Accession No. CBS 323.90.

Preferably the xylanase is a Family 11 type xylanase (formerly G-type xylanase), rather than a Family 10 (F-type) xylanase. Suitably position 37 is an aspartic acid (B) residue.

Preferably the modification is at a proline, cysterine, serine, alanine, leucine, valine, tyrosine, asparagine, threonine, arginine, glutamine, histidine and/or aspartic acid residue. Of these, modifications at Ser, Thr, Val, Ala, Asp, Asn, His, Tyr, Leu, Cys or Arg can be chosen. Optimally however the modification is at a serine, alanine, valine, tyrosine, aspartic acid or threonine residue. Especially preferred are modifications at a serine residue. Preferably the modification is at one or more of the positions 91, 92, 93, 94, 95, 98, 103, 108 or 155. Optimally the modification is at position 59 or 93 (these are both serine residues).

Suitably there are no modifications at the active cleft region. In other words, all modification(s) are outside the active cleft region. The active site in fungal xylanases includes residue 170. In bacterial xylanases the active cleft involves residues T10, F131, T172, G79 and E170. Preferably there are no modifications within 1, 2, 3, 4 or 5 residues either side of any of these active site residues.

Preferred modifications are at residues on the outside of the protein. This may thus not result in a major structural change to the enzyme. By changing these residues it has been found that one can alter the way in which the molecule behaves in its environment, that is to say for example thermostability. If one can thus avoid making modifications at residues at or towards the inside of the molecule then one may be able to avoid changing the activity of the molecule, in which case one may be able to increase thermostability without reducing activity. None of the prior art documents managed to achieve, at the same time, an increase in thermostability without decreasing activity. In addition, the prior art does not appreciate that this could be achieved by modifying the residues on the outside of the molecule, such as exposed serine residues, or other "outer" residues such as those within positions 90 to 160.

Of the serine residues that can be modified, these can include the residues at the positions 22, 27, 48, 55, 59, 61, 173, 179 or 183. Within the 90 to 160 region, the serine residues that can be modified include those at positions 93, 94, 97, 103, 106, 125, 136, 140 and 160.

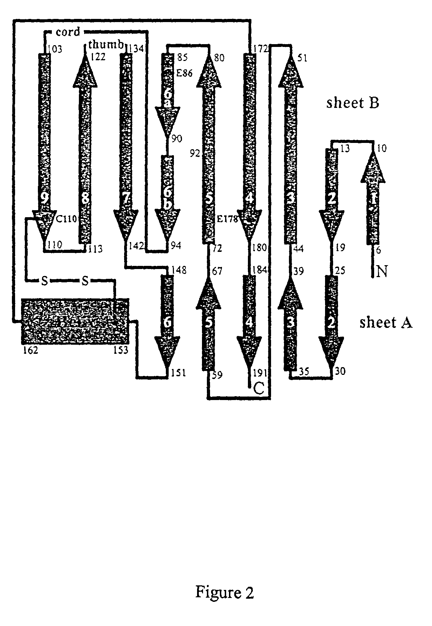

Preferred regions for modification therefore include residues at positions 91 to 104, 108, 113 to 117, 122 to 126, 136 to 140, 145 or 153 to 160. Preferably the modification is at one or more residues in the B7, B8 or B9 anti-parallel strand. The location of these strands within the xylanase can be seen from Figures 1, 2 and 6.

The most preferred modification is a single substitution of one amino acid residue for another. Although two or three of such modifications can be (and have been) made, good results have been found with single substitutions. Even with single point substitutions, the thermostability has been increased by more than 3, 4, 5 and 6 times, according to the modified residue.

Preferably the substituting residue is a Pro, Gly, Ala, Leu, Val, Ile, Phe, Tyr, Cys, Thr, Asn, His, Arg or Asp. Of these it is preferred that the substitution of the original residue is by a Leu, Cys, Thr, Asn, His, Arg or Asp residue. Particularly preferred positions for modification are 136, 103, 98, 93, 122, 95, 94, 92 and/or 91. Suitably one or more of positions 154 and 155 (both His) are substituted, or, if there are two neighbouring His residues, one of these is removed or substituted.

The invention encompasses (substantially) purified and/or isolated proteins. The proteins (or polypeptides, the terms are used interchangeably) of the invention may consist essentially of the amino acid sequence of SEQ ID No. 2 or 4 or of a variant of that sequence (other than the naturally occurring or wild-type sequence). The protein may also be encoded by a polynucleotide of the invention as described above.

The polypeptide may be mixed with carriers or diluents which will not interfere with the intended purpose and/of function of the polypeptide and still be regarded as substantially isolated. It will generally comprise the polypeptide in a preparation in which more than 20%, e.g. more than 30%, 40%, 50%, 80%, 90%, 95% or 99%, by weight of the polypeptide in the preparation is a polypeptide of the invention. Routine methods can be employed to purify and/or synthesise the proteins according to the invention¹⁶. For some formulations (e.g. for non-pharmaceutical uses) the amount of polypeptide present may be small, for example from 0.01 to 10%, such as from 0.1 to 5%, or 2% or even from 0.2 to 1%.

### Activity

A polypeptide of the invention can have one or more of the following features, namely it:
(1) possesses xylanase activity;
(2) has an optimum pH range of from 1.5 to 5.5, such as from 2 to 4, optimally from 2.5 to 3.5;
(3) has optimum activity at a temperature of from 30°C to 95°C, such as 60 to 90°C, optimally from 75 to 85°C;
(4) has a molecular weight of from 15 to 40 kDa, preferably from 20 to 30 kDa, optimally from 22 to 28 kDa or (deglycosylated) of from 10 to 30kDa, preferably from 15 to 25 kDa; and/or
(5) has an isoelectric point of from 2.5 to 4.5, such as 3.0 to 4.2, optimally from 3.3 to 3.9.

The polypeptide can have the activity of EC.3.2.1.8.

"Xylanase activity" is defined as the ability to cleave cellulose or xylan (for example as found in plants e.g. oat or barley). The activity thus allows cleavage of (1→4)-β-D-xylan, such as between adjacent xylopyranosyl terminal and/or non-terminal units. Preferably the cleavage occurs at a [xylopyranosyl(1-4)xylopyranosyl] linkage. The polypeptide may preferentially cleave in between two adjacent (e.g. non-substituted glucose) units. It can thus have endo activity (i.e. be an endoxylanase). The substrate polymer may or may not be substituted. Preferably the polypeptide will not have glucanase activity.

### Further Variants and Homologues

A polypeptide of the invention can comprise the amino acid sequence set out in SEQ ID No. 2 or 4 or a substantially homologous sequence, or a fragment of either sequence. It can have xylanase activity and the (or each) modification with respect to the naturally occurring xylanase. In particular, the polypeptide of the invention may comprise a protein with at least 70, at least 80, at least 90, at least 95, at least 98 or at least 99% sequence identity to SEQ. ID. No. 2, provided that it contains the modification(s) over the wild-type.

The polypeptide preferably has at least 70% sequence identity to the protein of SEQ ID No. 2 or 4, more preferably at least 80%, at least 90%, at least 95%, at east 97% or at least 99% sequence identity thereto, for example over a region of at least 60, at least 100, 150, 200 or 300 contiguous amino acids or over the full length of SEQ ID No. 2 or 4.

Polypeptides of the invention include fragments of the above mentioned full length polypeptides, including fragments of the sequence set out in SEQ ID No. 2 or 4. Such fragments typically retain activity as a xylanase. Fragments may be at least 100, 130 or 150 amino acids long and will include the (or each) modification.

Polypeptides of the invention can if necessary be produced by synthetic means although usually they will be made recombinantly as described below. They may be modified for example by the addition of histidine residues or a T7 tag to assist their identification or purification or by the addition of a signal sequence to promote their secretion from a cell.

Modifications may include the deletion, modification or addition of single amino acids or groups of amino acids within the protein sequence, preferably so that the peptide maintains the basic biological functionality of the xylanase. Modifications other than the modification(s) to increase thermostability can also be made.

As well as modifications to increase thermostability conservative substitutions may be made, for example according to the following Table. Amino acids in the same block in the second column and preferably in the same line in the third column may be substituted for each other. Preferably substitutions do not affect the folding or activity of the polypeptide.

| | | |
|---|---|---|
| ALIPHATIC | Non-polar | G A P |
| | | I L V |
| | Polar-uncharged | C S T M N Q |
| | Polar-charged | D E |
| | | K R |
| AROMATIC | | H F W Y |

Shorter polypeptide sequences are within the scope of the invention. For example, a peptide of at least 50 amino acids or up to 60, 70, 80, 100 or 150 amino acids in length is considered to fall within the scope of the invention as long as it demonstrates the basic biological functionality of the xylanase and contains one or more of the modifications that is not present in the naturally occuring xylanase. In particular, but not exclusively, this aspect of the invention encompasses the situation when the protein is a (modified) fragment of the complete protein sequence and may comprise or represent a xylan binding region or a xylan cleaving region.

### Alterations

Polypeptides of the invention may be chemically altered, e.g. post-translationally. For example, they may be glycosylated (one or more times, by the same or different sugars) or comprise modified amino acid residues. They may also be modified by the addition of histidine residues (to assist their purification) or by the addition of a signal sequence (to promote insertion into the cell membrane). The polypeptide may have one or more (N) amino- or (C) carboxyl-terminal extensions, such as an amino-terminal methionine residue, a small linker peptide of up to about 20-25 residues, or a (small) extension that facilitates purification, such as a poly-histidine or T7 tag, an antigenic epitope or a (e.g. maltose) binding domain¹⁴ (e.g. at the C-terminus). These extensions may or may not be added via a linker.

A polypeptide of the invention may be labelled with a revealing label. The revealing label may be any suitable label which allows the polypeptide to be detected. Suitable labels include radioisotopes, e.g. ¹²⁵I, ³⁵S, enzymes, antibodies, polynucleotides and linkers such as biotin.

The polypeptides may include non-naturally occurring amino acids. When the proteins or peptides are produced by synthetic means, such amino acids may be introduced during production. The proteins or peptides may also be modified following either synthetic or recombinant production.

The polypeptides of the invention may also be produced using, or comprise, (one or more) D-amino acids. In such cases the amino acid residues can be linked in reverse sequence in the C to N orientation or using the conventional N to C sequence as described in this application.

A number of side chain modifications are known in the art and may be made to the side chains of the proteins or peptides of the present invention. Such modifications include, for example, modifications of amino acids by reductive alkylation by reaction with an aldehyde followed by reduction with NaBH₄, amidination with methylacetimidate or acylation with acetic anhydride.

Amino acids important for thermostability or activity of the xylanases of the invention, and therefore potentially subject to substitution, may be identified and modified according to procedures known in the art, such as site-directed mutagenesis or alanine-scanning mutagenesis²¹. In the latter technique mutations are introduced at every residue in the molecule, and the resultant mutant molecules are tested for biological activity (e.g. xylanase activity) to identify amino acid residues that are critical to the activity of the molecule. Sites of enzyme-substrate interaction can also be determined by analysis of crystal structure as determined by such techniques as nuclear magnetic resonance, crystallography or photo-affinity labelling^{22,23,24} or molecular modelling.

The use of yeast and fungal host cells is expected to provide for such post-translational modifications (e.g. proteolytic processing, myristilation, glycosylation, truncation, and tyrosine, serine or threonine phosphorylation) as may be needed to confer optimal biological activity on recombinant expression products of the invention.

Polypeptides of the invention may be provided in a form such that they are outside their natural cellular environment. Thus, they may be substantially isolated or purified, as discussed above, or in a cell in which they do not occur in nature, e.g. a cell of other fungal species, animals, yeast or bacteria.

### B. Polynucleotides.

The present invention provides an (e.g. isolated and/or purified) polynucleotide encoding a protein of the invention. The present invention thus provides a polynucleotide encoding either a xylanase whose amino acid sequence is set out in SEQ ID No. 1 or 3 or a protein as defined in the first aspect. The present invention further provides a polynucleotide encoding a protein having substantial amino acid sequence homology to the amino acid sequence set out in SEQ ID No. 2 or 4, provided that the modification (over the wild type) is present. Also included is a polynucleotide selected from:
(a) a polynucleotide comprising the nucleotide sequence set out in SEQ ID No. 1 or 3, or the complement thereof;
(b) a polynucleotide comprising a nucleotide sequence capable of (e.g. selectively) hybridising to a nucleotide sequence set out in SEQ ID No. 1 or 3, or a fragment thereof;
(c) a polynucleotide comprising a nucleotide sequence capable of (e.g. selectively) hybridising to the complement of the nucleotide sequence set out in SEQ ID No. 1 or 3, or a fragment thereof; and/or
(d) a polynucleotide comprising a polynucleotide sequence that is degenerate as a result of the genetic code to a polynucleotide defined in (a), (b) or (c).

Polynucleotides in (b) and (c) can contain the change(s) corresponding to the modification(s) in the protein.

A polynucleotide of the invention also includes a polynucleotide which:
(a) encodes a protein of the first aspect having xylanase activity, which polynucleotide is:
   (1) the coding sequence of SEQ ID No. 1 or 3;
   (2) a sequence which hybridises selectively to the complement of sequence defined in (1) and includes one or more bases corresponding to the modification(s) in the protein of the first aspect; or
   (3) a sequence that is degenerate as a result of the genetic code with respect to a sequence defined in (1) or (2); or
(b) is a sequence complementary to a polynucleotide defined in (a).

### Hybridisable sequences

The term "capable of hybridizing" means that the target polynucleotide of the invention can hybridize to the nucleic acid used as a probe (for example the nucleotide sequence set out in SEQ. ID No. 1 or 3, or a fragment thereof or the complement thereof) at a level significantly above background. The nucleotide sequence may be RNA or DNA and thus includes genomic DNA, synthetic DNA or cDNA. Preferably the nucleotide sequence is a DNA sequence and most preferably, a cDNA sequence. Polynucleotides of the invention can be synthesized according to methods well known in the art²⁸.

A polynucleotide of the invention can hybridize to the coding sequence or the complement of the coding sequence of SEQ ID No.1 at a level significantly above background. Background hybridization may occur, for example, because of other cDNAs present in a cDNA library. The signal level (eg. generated by the interaction between a polynucleotide of the invention and the coding sequence or complement of the coding sequence of SEQ ID No. 1 or 3) is typically at least 10 fold, preferably at least 100 fold, as intense as interactions between other polynucleotides and the coding sequence of SEQ ID No. 1 or 3. The intensity of interaction may be measured, for example, by radiolabelling the probe, e.g. with ³²P. Selective hybridization may typically be achieved using conditions of low stringency (0.3M sodium chloride and 0.03M sodium citrate at about 40°C), medium stringency (for example, 0.3M sodium chloride and 0.03M sodium citrate at about 50°C) or high stringency (for example, 0.3M sodium chloride and 0.03M sodium citrate at about 60°C). Hybridization may be carried out under any suitable conditions known in the art' and, as a guide, low stringency can be 2 x SSC at 55°C, medium stringency can be 0.5 to 1.0 x SSC at 60°C and high stringency can be 0.1 or 0.2 x SSC at 60°C or higher (e.g. at 68°C), all at 0.5% SDS.

### Primers, Probes and Oligonucleotide - directed mutagenesis

Polynucleotides of the invention include and may be used to perform site-directed mutagenesis, as a primer, e.g. a PCR primer, a primer for an alternative amplification reaction, a probe, or the polynucleotides may be cloned into vectors. Such primers, probes and other fragments will be at least or up to 20, for example at least 25, 30 or 40 nucleotides in length. They will typically be up to 40, 50, 60, 70, 100, 150, 200 or 300 nucleotides in length, or even up to a few nucleotides (such as 5 or 10 nucleotides) short of the coding sequence of SEQ ID No. 1 or 3.

In general, primers will be produced by synthetic means, involving a step-wise manufacture of the desired nucleic acid sequence one nucleotide at a time. Techniques for accomplishing this using automated techniques are readily available in the art. Examples of primers of the invention are set out in SEQ ID Nos 5 to 101.

Longer polynucleotides will generally be produced using recombinant means, for example using PCR (polymerase chain reaction) cloning techniques. This will involve making one or more oligonucleotides (e.g. of about 15-30 nucleotides) to a region of the which it is desired to alter, bringing the primers into contact with obtained from a (e.g yeast, bacterial, plant, prokaryotic or fungal) cell, preferably of an *Aspergillus* strain. The oligo will have one or more mismatches to the natural sequence to effect the change at one or more bases. The primer can then be elongated by DNA polymerase.

One can also perform a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector.

The polynucleotides or primers may carry a revealing label, e.g. a radioactive or nonradioactive label. Suitable labels include radioisotopes such as ³²P or ³⁵S, enzyme labels, or other protein labels such as biotin. Such labels may be added to polynucleotides or primers of the invention and may be detected using techniques known *per se.*

### Alterations

Polynucleotides of the invention may comprise DNA or RNA. They may be single or double stranded. They may also be polynucleotides which include within them one or more synthetic or modified nucleotides. A number of different types of modifications to polynucleotides are known in the art. These include methylphosphonate and phosphorothioate backbones and/or addition of acridine or polylysine chains at the 3' and/or 5' ends of the molecule. For the purposes of the present invention, it is to be understood that the polynucleotides described herein may be modified by any method available in the art.

It is to be understood that skilled persons may, using routine techniques, make nucleotide substitutions that do not affect the protein sequence encoded by the polynucleotides of the invention to reflect the codon usage of any particular host organism, for example in which the polypeptides of the invention are to be expressed.

The coding sequence of SEQ ID No. 1 may be modified by nucleotide substitutions, for example from or up to 1, 2 or 3 to 10, 25, 50 or 100 substitutions. The polynucleotide may alternatively or additionally be modified by one or more insertions and/or deletions and/or by an extension at either or both ends. The modified polynucleotide generally encodes a protein which has xylanase activity. Degenerate substitutions may be made and/or substitutions may be made which would result in a conservative amino acid substitution when the modified sequence is translated, for example as discussed with reference to proteins later.

### C. Recombinant Aspects

The invention also provides vectors comprising a polynucleotide of the invention, including cloning and expression vectors, and methods of growing, transforming or transfecting such vectors in a suitable host cell, for example under conditions in which expression of a polypeptide of the invention occurs. Provided also are host cells comprising a polynucleotide or vector of the invention wherein the polynucleotide is heterologous to the genome of the host cell. The term "heterologous", usually with respect to the host cell, means that the polynucleotide does not naturally occur in the genome of the host cell or that the polypeptide is not naturally produced by that cell.

Preferably, the host cell is a yeast cell, for example a yeast cell of the genus *Kluyveromyces* or *Saccharomyces* or a fungal cell, for example of the genus *Aspergillus.*

Polynucleotides of the invention can be incorporated into a recombinant replicable vector, for example a cloning or expression vector. The vector may be used to replicate the nucleic acid in a compatible host cell. Thus in a further embodiment, the invention provides a method of making polynucleotides of the invention by introducing a polynucleotide of the invention into a replicable vector, introducing the vector into a compatible host cell, and growing the host cell under conditions which bring about replication of the vector. The vector may be recovered from the host cell. Suitable host cells are described below in connection with expression vectors.

### Vectors

The polynucleotide of the invention may inserted into an expression cassette. The vector into which the expression cassette or polynucleotide of the invention is inserted may be any vector which may conveniently be subjected to recombinant DNA procedures, and the choice of the vector will often depend on the host cell into which it is to be introduced. Thus, the vector may be an autonomously replicating vector, i.e. a vector which exists as an extra-chromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated.

Preferably, a polynucleotide of the invention in a vector is operably linked to a regulatory sequence which is capable of providing for the expression of the coding sequence by the host cell, i.e. the vector is an expression vector. The term "operably linked" refers to a juxtaposition wherein the components described are in a relationship permitting them to function in their intended manner. A regulatory sequence such as a promoter, enhancer or other expression regulation signal "operably linked" to a coding sequence is positioned in such a way that expression of the coding sequence is achieved under condition compatible with the control sequences.

The vector may be a plasmid, cosmid, virus or phage vector, usually provided with an origin of replication, optionally a promoter for the expression of the polynucleotide and optionally an enhancer and/or a regulator of the promoter. A terminator sequence may be present, as may be a polyadenylation sequence. The vector may contain one or more selectable marker genes, for example an ampicillin resistance gene (in the case of a bacterial plasmid) or a neomycin resistance gene (for a mammalian vector). Vectors may be used *in vitro,* for example for the production of RNA or used to transfect or transform a host cell. They may comprise two or more polynucleotides of the invention, for example for overexpression.

The DNA sequence encoding the polypeptide is preferably introduced into a suitable host as part of an expression cassette (or construct) in which the DNA sequence is operably linked to expression signals which are capable of directing expression of the DNA sequence in the host cells. For transformation of the suitable host with the expression construct transformation procedures are available which are well known to the skilled person^{25,26}. The expression construct can be used for transformation of the host as part of a vector carrying a selectable marker, or the expression construct may be co-transformed as a separate molecule together with the vector carrying a selectable marker. The vector may comprise one or more selectable marker genes.

Preferred selectable markers^{27,28} include but are not limited to those that complement a defect in the host cell or confer resistance to a drug. They include e.g. versatile marker genes that can be used for transformation of most filamentous fungi and yeasts such as acetamidase genes or cDNAs (the *amdS, niaD*, *facA* genes or cDNAs from *A.nidulans*, *A.oryzae,* or *A.niger*), or genes providing resistance to antibiotics like G418, hygromycin, bleomycin, kanamycin, phleomycin or benomyl resistance (benA). Alternatively, specific selection markers can be used such as auxotrophic markers which require corresponding mutant host strains: e.g. *URA3* (from *S.cerevisiae* or analogous genes from other yeasts), *pyrG* or *pyrA* (from *A. nidulans* or *A. niger), argB* (from *A.nidulans* or *A.niger*) or *trpC*. In a preferred embodiment the selection marker is deleted from the transformed host cell after introduction of the expression construct so as to obtain transformed host cells capable of producing the polypeptide which are free of selection marker genes^{29,30}.

Other markers include ATP synthetase, subunit 9 *(oli*C), orotidine-5'-phosphate-decarboxylase (*pvr*A), the bacterial G418 resistance gene (this may also be used in yeast, but not in fungi), the ampicillin resistance gene (*E. coli),* the neomycin resistance gene *(Bacillus)* and the *E. coli uid* A gene, coding for β-glucuronidase (GUS). Vectors may be used *in vitro,* for example for the production of RNA or used to transfect or transform a host cell.

For most filamentous fungi and yeast, the vector or expression construct is preferably integrated in the genome of the host cell in order to obtain stable transformants. However, for certain yeasts also suitable episomal vectors are available into which the expression construct can be incorporated for stable and high level expression, examples thereof include vectors derived from the 2µ and pKD1 plasmids of *Saccharomyces* and *Kluyveromyces,* respectively, or vectors containing an AMA sequence (e.g. AMA1 from *Aspergillus*^{25,31}). In case the expression constructs are integrated in the host cells genome, the constructs are either integrated at random loci in the genome, or at predetermined target loci using homologous recombination, in which case the target loci preferably comprise a highly expressed gene. A highly expressed gene is a gene whose mRNA can make up at least 0.01% (w/w) of the total cellular mRNA, e.g. under induced conditions, or alternatively, a gene whose gene product can make up at least 0.2% (w/w) of the total cellular protein, or, in case of a secreted gene product, can be secreted to a level of at least 0.05g/l. A number of examples of suitable highly expressed genes are provided below.

A vector or expression construct for a given host cell may comprise the following elements operably linked to each other in a consecutive order from the 5'-end to 3'-end relative to the coding strand of the sequence encoding the polypeptide of the first aspect:
(1) a promoter sequence capable of directing transcription of the DNA sequence encoding the polypeptide in the given host cell;
(2) optionally, a signal sequence capable of directing secretion of the polypeptide from the given host cell into a culture medium;
(3) a DNA sequence encoding a mature and preferably active form of the polypeptide; and preferably also
(4) a transcription termination region (terminator) capable of terminating transcription downstream of the DNA sequence encoding the polypeptide.

Downstream of the DNA sequence encoding the polypeptide there may be a 3' untranslated region containing one or more transcription termination sites (e.g. a terminator). The origin of the terminator is less critical. The terminator can e.g. be native to the DNA sequence encoding the polypeptide. However, preferably a yeast terminator is used in yeast host cells and a filamentous fungal terminator is used in filamentous fungal host cells. More preferably, the terminator is endogenous to the host cell (in which the DNA sequence encoding the polypeptide is to be expressed).

Enhanced expression of the polynucleotide encoding the polypeptide of the invention may also be achieved by the selection of heterologous regulatory regions, e.g. promoter, secretion leader and/or terminator regions, which may serve to increase expression and, if desired, secretion levels of the protein of interest from the expression host and/or to provide for the inducible control of the expression of the polypeptide of the invention.

Aside from the promoter native to the gene encoding the polypeptide of the invention, other promoters may be used to direct expression of the polypeptide of the invention. The promoter may be selected for its efficiency in directing the expression of the polypeptide of the invention in the desired expression host.

Promoters/enhancers and other expression regulation signals may be selected to be compatible with the host cell for which the expression vector is designed. For example prokaryotic promoters may be used, in particular those suitable for use in *E.coli* strains. When expression is carried out in mammalian cells, mammalian promoters may be used. Tissues-specific promoters, for example hepatocyte cell-specific promoters, may also be used. Viral promoters may also be used, for example the Moloney murine leukaemia virus long terminal repeat (MMLV LTR), promoter rous sarcoma virus (RSV) LTR promoter, SV40 (e.g. large T antigen) promoter, human cytomegalovirus (CMV) IE promoter, herpes simplex virus promoters or adenovirus promoters, HSV promoters such as the HSV IE promoters), or HPV promoters, particularly the HPV upstream regulatory region (URR). Yeast promoters include *S. cerevisiae* GAL4 and ADH promoters, the *S. pombe nmt* 1 and *adh* promoter. Mammalian promoters include the metallothionein promoter which may be induced in response to heavy metals such as cadmium and β-actin promoters. Tissue-specific promoters, in particular endothelial or neuronal cell specific promoters (for example the DDAHI and DDAHII promoters), are especially preferred.

A variety of promoters^{27,28} can be used that are capable of directing transcription in the host cells of the invention. Preferably the promoter sequence is derived from a highly expressed gene as previously defined. Examples of preferred highly expressed genes from which promoters are preferably derived and/or which are comprised in preferred predetermined target loci for integration of expression constructs, include but are not limited to genes encoding glycolytic enzymes such as triose-phosphate isomerases (TPI), glyceraldehyde-phosphate dehydrogenases (GAPDH), phosphoglycerate kinases (PGK), pyruvate kinases (PYK), alcohol dehydrogenases (ADH), as well as genes encoding amylases, glucoamylases, proteases, glucanases, cellobiohydrolases, β-galactosidases, alcohol (methanol) oxidases, elongation factors and ribosomal proteins. Specific examples of suitable highly expressed genes include e.g. the *LAC4* gene from *Kluyveromyces* sp., the methanol oxidase genes (*AOX* and *MOX*) from *Hansenula* and *Pichia*, respectively, the glucoamylase (*glaA*) genes from *A.niger* and *A.awamori*, the *A.oryzae* TAKA-amylase gene, the *A.nidulans gpdA* gene and the *T.reesei* cellobiohydrolase genes.

Examples of strong constitutive and/or inducible promoters which are preferred for use in fungal expression hosts^{27,28} are those which are obtainable from the fungal genes for xylanase (*xln*A), phytase, ATP-synthetase, subunit 9 *(oli*C), triose phosphate isomerase (*tpi*), alcohol dehydrogenase (*Adh*A), α-amylase (*amy*), amyloglucosidase (AG - from the *gla*A gene), acetamidase (*amd*S) and glyceraldehyde-3-phosphate dehydrogenase (*gpd*) promoters.

Examples of strong yeast promoters are those obtainable from the genes for alcohol dehydrogenase, lactase, 3-phosphoglycerate kinase and triosephosphate isomerase.

Examples of strong bacterial promoters are the α-amylase and *SPo2* promoters as well as promoters from extracellular protease genes.

Promoters suitable for plant cells include napaline synthase (nos), octopine synthase (ocs), mannopine synthase (mas), ribulose small subunit (rubisco ssu), histone, rice actin, phaseolin, cauliflower mosaic virus (CMV) 35S and 19S and circovirus promoters. All these promoters are readily available in the art.

The vector may further include sequences flanking the polynucleotide giving rise to RNA which comprise sequences homologous to eukaryotic genomic sequences, preferably mammalian genomic sequences, or viral genomic sequences. This will allow the introduction of the polynucleotides of the invention into the genome of eukaryotic cells or viruses by homologous recombination. In particular, a plasmid vector comprising the expression cassette flanked by viral sequences can be used to prepare a viral vector suitable for delivering the polynucleotides of the invention to a mammalian cell. Other examples of suitable viral vectors include herpes simplex viral vectors^{32,33} and retroviruses, including lentiviruses, adenoviruses, adeno-associated viruses and HPV viruses (such as HPV-16 or HPV-18). Gene transfer techniques using these viruses are known to those skilled in the art. Retrovirus vectors for example may be used to stably integrate the polynucleotide giving rise to the antisense RNA into the host genome. Replication-defective adenovirus vectors by contrast remain episomal and therefore allow transient expression.

The vector may contain a polynucleotide of the invention oriented in an antisense direction to provide for the production of antisense RNA. This may be used to reduce, if desirable, the levels of expression of the polypeptide.

### Host cells and Expression

In a further aspect the invention provides a process for preparing a polypeptide according to the invention which comprises cultivating a host cell (e.g. transformed or transfected with an expression vector as described above) under conditions to provide for expression (by the vector) of a coding sequence encoding the polypeptide, and optionally recovering the expressed polypeptide. Polynucleotides of the invention can be incorporated into a recombinant replicable vector, e.g. an expression vector. The vector may be used to replicate the nucleic acid in a compatible host cell. Thus in a further embodiment, the invention provides a method of making a polynucleotide of the invention by introducing a polynucleotide of the invention into a replicable vector, introducing the vector into a compatible host cell, and growing the host cell under conditions which bring about the replication of the vector. The vector may be recovered from the host cell. Suitable host cells include bacteria such as *E. coli,* yeast (e.g. *Kluyveromyces*), mammalian cell lines and other eukaryotic cell lines, for example insect cells such as Sf9 cells and (e.g. filamentous) fungal cells.

Preferably the polypeptide is produced as a secreted protein in which case the DNA sequence encoding a mature form of the polypeptide in the expression construct is operably linked to a DNA sequence encoding a signal sequence. Preferably the signal sequence is native (homologous) to the DNA sequence encoding the polypeptide. Alternatively the signal sequence is foreign (heterologous) to the DNA sequence encoding the polypeptide, in which case the signal sequence is preferably endogenous to the host cell in which the DNA sequence is expressed. Examples of suitable signal sequences for yeast host cells are the signal sequences derived from yeast α-factor genes. Similarly, a suitable signal sequence for filamentous fungal host cells is e.g. a signal sequence derived from a filamentous fungal amyloglucosidase (AG) gene, e.g. the *A.niger glaA* gene. This may be used in combination with the amyloglucosidase (also called (gluco)amylase) promoter itself, as well as in combination with other promoters. Hybrid signal sequences may also be used with the context of the present invention.

Preferred heterologous secretion leader sequences are those originating from the fungal amyloglucosidase (AG) gene (*gla*A - both 18 and 24 amino acid versions e.g. from *Aspergillus),* the α-factor gene (yeasts e.g. *Saccharomyces* and *Kluyveromyces)* or the α-amylase gene *(Bacillus).*

The vectors may be transformed or transfected into a suitable host cell as described above to provide for expression of a polypeptide of the invention. This process may comprise culturing a host cell transformed with an expression vector as described above under conditions to provide for expression by the vector of a coding sequence encoding the polypeptide.

A further aspect of the invention thus provides host cells transformed or transfected with or comprising a polynucleotide or vector of the invention. Preferably the polynucleotide is carried in a vector for the replication and expression of the polynucleotide. The cells will be chosen to be compatible with the said vector and may for example be prokaryotic (for example bacterial), fungal, yeast or plant cells.

A heterologous host may also be chosen wherein the polypeptide of the invention is produced in a form which is substantially free from other cellulose-degrading enzymes. This may be achieved by choosing a host which does not normally produce such enzymes such as *Kluyveromyces lactis*.

The invention encompasses processes for the production of the polypeptide of the invention by means of recombinant expression of a DNA sequence encoding the polypeptide. For this purpose the DNA sequence of the invention can be used for gene amplification and/or exchange of expression signals, such as promoters, secretion signal sequences, in order to allow economic production of the polypeptide in a suitable homologous or heterologous host cell. A homologous host cell is herein defined as a host cell which is of the same species or which is a variant within the same species as the species from which the DNA sequence is derived.

Suitable host cells are preferably prokaryotic microorganisms such as bacteria, or more preferably eukaryotic organisms, for example fungi, such as yeasts or filamentous fungi, or plant cells. In general, yeast cells are preferred over fungal cells because they are easier to manipulate. However, some proteins are either poorly secreted from yeasts, or in some cases are not processed properly (e.g. hyperglycosylation in yeast). In these instances, a fungal host organism should be selected.

The host cell may over-express the polypeptide, and techniques for engineering over-expression are well known²⁵. The host may thus have two or more copies of the encoding polynucleotide (and the vector may thus have two or more copies accordingly).

Bacteria from the genus *Bacillus* are very suitable as heterologous hosts because of their capability to secrete proteins into the culture medium. Other bacteria suitable as hosts are those from the genera *Streptomyces* and *Pseudomonas*. A preferred yeast host cell for the expression of the DNA sequence encoding the polypeptide is of the genera *Saccharomyces, Kluyveromyces, Hansenula, Pichia, Yarrowia*, and *Schizosaccharomyces*. More preferably a yeast host cell is selected from the group consisting of the species *Saccharomyces cerevisiae, Kluyveromyces lactis* (also known as *Kluyveromyces marxianus* var. *lactis*), *Hansenula polymorpha, Pichia pastoris, Yarrowia lipolytica*,and *Schizosaccharomyces pombe.*

Most preferred are, however, (e.g. filamentous) fungal host cells. Preferred filamentous fungal host cells are selected from the group consisting of the genera *Aspergillus, Trichoderma, Fusarium*, *Disporotrichum, Penicillium, Acremonium, Neurospora, Thermoascus, Myceliophtora, Sporotrichum, Thielavia,* and *Talaromyces.* More preferably a filamentous fungal host cell is of the species *Aspergillus oyzae*, *Aspergillus sojae, Aspergillus nidulans*, or a species from the *Aspergillus niger* Group (as defined by Raper and Fennell, The Genus *Aspergillus,* The Williams & Wilkins Company, Baltimore, pp 293-344, 1965). These include but are not limited to *Aspergillus niger, Aspergillus awamori, Aspergillus tubingensis, Aspergillus aculeatus, Aspergillus foetidus*, *Aspergillus nidulans, Aspergillus japonicus, Aspergillus oryzae* and *Aspergillus ficuum*, and further consisting of the species *Trichoderma reesei, Fusarium graminearum, Penicillium chrysogenum, Acremonium alabamense, Neurospora crassa, Myceliophtora thermophilum, Sporotrichum cellulophilum, Disporotrichum dimorphosporum* and *Thielavia terrestris.*

Examples of preferred expression hosts within the scope of the present invention are fungi such as *Aspergillus* species^{34,35} and *Trichoderma* species; bacteria such as *Bacillus* species^{36,37} e.g. *Bacillus subtilis, Bacillus lichenfiormis, Bacillus amyloliquefaciens, Pseudomonas* species; and yeasts such as *Kluyveromyces* species³⁸ e.g. *Kluyveromyces lactis*³⁹ and *Saccharomyces* species, e.g. *Saccharomyces cerevisiae*.

Host cells according to the invention include plant cells, and the invention therefore extends to transgenic organisms, such as plants and parts thereof, which contain one or more cells of the invention. The cells may heterologously express the polypeptide of the invention or may heterologously contain one or more of the polynucleotides of the invention. The transgenic (or genetically modified) plant may therefore have inserted (e.g. stably) into its genome a sequence encoding one or more of the polypeptides of the invention. The transformation of plant cells can be performed using known techniques, for example using a Ti or a Ri plasmid from *Agrobacterium tumefaciens*. The plasmid (or vector) may thus contain sequences necessary to infect a plant, and derivatives of the Ti and/or Ri plasmids may be employed.

Alternatively direct infection of a part of a plant, such as a leaf, root or stem can be effected. In this technique the plant to be infected can be wounded, for example by cutting the plant with a razor or puncturing the plant with a needle or rubbing the plant with an abrasive. The wound is then innoculated with the *Agrobacterium*. The plant or plant part can then be grown on a suitable culture medium and allowed to develop into a mature plant. Regeneration of transformed cells into genetically modified plants can be achieved by using known techniques, for example by selecting transformed shoots using an antibiotic and by sub-culturing the shoots on a medium containing the appropriate nutrients, plant hormones and the like.⁴⁰

### Culture of host cells and recombinant production

The invention also includes cells that have been modified to express the polypeptides comprising the modified xylanse. Such cells include transient, or preferably stable higher eukaryotic cell lines, such as mammalian cells or insect cells, lower eukaryotic cells, such as yeast and (e.g. filamentous) fungal cells or prokaryotic cells such as bacterial cells.

It is also possible for the proteins of the invention to be transiently expressed in a cell line or on a membrane, such as for example in a baculovirus expression system. Such systems, which are adapted to express the proteins according to the invention, are also included within the scope of the present invention.

According to the present invention, the production of the polypeptide of the invention can be effected by the culturing of microbial expression hosts, which have been transformed with one or more polynucleotides of the present invention, in a conventional nutrient fermentation medium.

The recombinant host cells according to the invention may be cultured using procedures known in the art. For each combination of a promoter and a host cell, culture condition are available which are conducive to the expression the DNA sequence encoding the polypeptide. After reaching the desired cell density or titre of the polypeptide the culture is stopped and the polypeptide is recovered using known procedures.

The fermentation medium can comprise a known culture medium containing a carbon source (e.g. glucose, maltose, molasses, etc.), a nitrogen source (e.g. ammonium sulphate, ammonium nitrate, ammonium chloride, etc.), an organic nitrogen source (e.g. yeast extract, malt extract, peptone, etc.) and inorganic nutrient sources (e.g. phosphate, magnesium, potassium, zinc, iron, etc.). Optionally, an inducer (e.g. cellulose, pectin, maltose, maltodextrin or xylogalacturonan) may be included.

The selection of the appropriate medium may be based on the choice of expression host and/or based on the regulatory requirements of the expression construct. Such media are known to those skilled in the art. The medium may, if desired, contain additional components favouring the transformed expression hosts over other potentially contaminating microorganisms.

The fermentation can be performed over a period of 0.5-30 days. It may be a batch, continuous or fed-batch process, suitably at a temperature in the range of between 0 and 45°C and, for example, at a pH between 2 and 10. Preferred fermentation conditions are a temperature in the range of between 20 and 37°C and/or a pH between 3 and 9. The appropriate conditions are usually selected based on the choice of the expression host and the protein to be expressed.

After fermentation, if necessary, the cells can be removed from the fermentation broth by means of centrifugation or filtration. After fermentation has stopped or after removal of the cells, the polypeptide of the invention may then be recovered and, if desired, purified and isolated by conventional means.

### D. Uses of the modified xylanase and methods of processing plant or cellulose (e.g. xylan)-containing materials

The polypeptides of the invention which possess xylanase activity may be used to treat fungal or plant material including plant pulp and plant extracts. For example, they may be used to treat cereals, vegetables, fruits or extracts thereof. Conveniently the polypeptide of the invention is combined with suitable (solid or liquid) carriers or diluents including buffers to produce a composition/ enzyme preparation. The polypeptide may be attached to or mixed with a carrier, e.g. immobilized on a solid carrier. Thus the present invention provides in a further aspect a composition comprising a polypeptide of the invention. This may be in a form suitable for packaging, transport and/or storage, preferably where the xylanase activity is retained. Compositions may be of paste, liquid, emulsion, powder, flake, granulate, pellet or other extrudate form.

The composition may further comprise additional ingredients such as one or more enzymes, for example pectinases, including endo-arabinanase and rhamnogalacturonase, cellulases, (other) xylanases, galacturonases, mannanases and/or xyloglucanases. The polypeptide is typically stably formulated either in liquid or dry form. Typically, the product is made as a composition which will optionally include, for example, a stabilising buffer and/or preservative. The compositions may also include other enzymes capable of digesting plant material or cellulose, for example other cellulases, e.g. (β-D-)glucanases. For certain applications, immobilization of the enzyme on a solid matrix or incorporation on or into solid carrier particles may be preferred. The composition may also include a variety of other plant material-degrading enzymes, for example cellulases and other pectinases.

The polypeptides and compositions of the invention may therefore be used in a method of processing plant material to degrade or modify the cellulose constituents (e.g. xylan) of the cell walls of the plant or fungal material. Thus in a further aspect, the present invention provides a method of degrading or modifying a plant cell which method comprises contacting the plant or fungal cell with a polypeptide or composition of the invention.

The invention also provides a method of processing a plant material which method comprises contacting the plant material with a polypeptide or composition of the invention to degrade or modify the cellulose in the (plant) material. Preferably the plant material is a plant pulp or plant extract, such as juices.

In particular, the degradation preferably comprises cleaving of xylan subunits of a cellulose component of the plant cell wall. The plant material is preferably a cereal, vegetable, fruit or vegetable or fruit pulp or extract. The present invention further provides a processed plant material obtainable by contacting a plant material with a polypeptide or composition of the invention.

The present invention also provides a method for reducing the viscosity of a plant extract which method comprises contacting the plant extract with a polypeptide or composition of the invention in an amount effective in degrading cellulose (or xylan) contained in the plant extract.

Plant and cellulose-containing materials include plant pulp, parts of plants and plant extracts. In the context of this invention an extract from a plant material is any substance which can be derived from plant material by extraction (mechanical and/or chemical), processing or by other separation techniques. The extract may be juice, nectar, base, or concentrates made thereof. The plant material may comprise or be derived from vegetables, e.g., carrots, celery, onions, legumes or leguminous plants (soy, soybean, peas) or fruit, e.g., pome or seed fruit (apples, pears, quince etc.), grapes, tomatoes, citrus (orange, lemon, lime, mandarin), melons, prunes, cherries, black currants, redcurrants, raspberries, strawberries, cranberries, pineapple and other tropical fruits, trees and parts thereof (e.g. pollen, from pine trees), or cereal (oats, barley, wheat, maize, rice). The material (to be hydrolysed) may also be agricultural residues, such as sugar beet pulp, corn cobs, wheat straw, (ground) nutshells, or recyclable materials, e.g. (waste) paper.

The polypeptides of the invention can thus be used to treat plant material including plant pulp and plant extracts. They may also be used to treat liquid or solid foodstuffs or edible foodstuff ingredients, or be used in the extraction of coffee, plant oils, starch or as a thickener in foods.

Typically, the polypeptides of the invention are used as a composition/ enzyme preparation as described above. The composition will generally be added to plant pulp obtainable by, for example mechanical processing such as crushing or milling plant material. Incubation of the composition with the plant will typically be carried out for at time of from 10 minutes to 5 hours, such as 30 minutes to 2 hours, preferably for about 1 hour. The processing temperature is preferably 10-55°C, e.g. from 15 to 25°C, optimally about 20°C and one can use 10-300g, preferably 30-70g, optimally about 50g of enzyme per ton of material to be treated. All the enzyme(s) or their compositions used may be added sequentially or at the same time to the plant pulp. Depending on the composition of the enzyme preparation the plant material may first be macerated (e.g. to a purée) or liquefied. Using the polypeptides of the invention processing parameters such as the yield of the extraction, viscosity of the extract and/or quality of the extract can be improved.

Alternatively, or in addition to the above, a polypeptide of the invention may be added to the raw juice obtained from pressing or liquefying the plant pulp. Treatment of the raw juice will be carried out in a similar manner to the plant pulp in respect of dosage, temperature and holding time. Again, other enzymes such as those discussed previously may be included. Typical incubation conditions are as described in the previous paragraph. Once the raw juice has been incubated with the polypeptides of the invention, the juice is then centrifuged or (ultra) filtered to produce the final product.

After treatment with the polypeptide of the invention the (end) product can be heat treated, e.g. at 85°C for a time of from 1 minute to 1 hour, under conditions to partially or fully inactivate the polypeptide(s) of the invention.

A composition containing a polypeptide of the invention may also be used during the preparation of fruit or vegetable purees.

The polypeptide of the invention may also be used in brewing, wine making, distilling or baking. It may therefore used in the preparation of alcoholic beverages such as wine and beer. For example it may improve the filterability or clarity (of beers, wort or wine). The protein may assist in the removal of dissolved organic substances from broth or culture media, for example where distillery waste from organic origin is bioconverted into microbial biomass. The xylanase can improve filterability and/or reduce viscosity in glucose syrups, such as from cereals produced by liquefaction (e.g. with α-amylase).

In baking the polypeptide may improve the dough structure, modify its stickiness or suppleness, improve the loaf volume and/or crumb structure or impart better textural characteristics such as break, shread or crumb quality.

The polypeptides find use in a number of industrial areas due to their xylanase activity. These can include not only alcohol production, but also in biomethanation, in bread making and in baking, in dental hygiene (for example dental or oral compositions), in the treatment or manufacture of leather, in the manufacture of paper, in pharmaceuticals, in tea, in the preparation or treatment of textiles, and in the treatment of waste. One aspect of the invention is therefore a food or foodstuff comprising the polypeptide, such as an alcoholic beverage, bread, dough or tea. The polypeptide may be formulated into a suitable compositions for any of these uses. The polypeptide may be present in an aqueous composition (eg. hot water), preferably with one or more fungicides, in order to treat plant material (eg. bulbs), especially to control parasitic insects, mites and nematodes.
As the polypeptides of the invention can degrade xylan they may be added to foods or foodstuffs (for example by consumption by humans). The invention also includes pharmaceutical and veterinary compositions that comprise the polypeptide of the invention and the pharmaceutically or veterinarily acceptable carrier.

Polypeptides of the invention may also display anti-fungal activity. They may be able to degrade fungal cell walls, and thus can be employed for fungal cell wall lysis, in order to open the cells. This may release intracellular proteins. In such a way the polypeptides may be used to prepare yeast and/or fungal extracts.

### E. Animal Feeds

The invention additionally relates to foodstuffs or an animal feed composition or additive comprising one or more polypeptides of the invention. The polypeptide may be present in the feed at a concentration different from its natural concentration. Preferred amounts are from 0.1 to 100, such as 0.5 to 50, preferably 1 to 10, mg per kg feed.

The invention also relates to a process for the preparation of an animal feed composition, the process comprising adding to one or more edible feed substance(s) or ingredient(s), suitably containing xylan, a polypeptide of the invention. The polypeptides can be added to the animal feed composition separately from the feed substances or ingredients, individually or in combination with other feed additives. The polypeptide can be an integral part of one of the feed substances or ingredients.

The polypeptides of the invention may also be added to animal feeds rich in cellulose to improve the breakdown of the plant cell wall leading to improved utilisation of the plant nutrients by the animal. The polypeptides of the invention may be added to the feed or silage if pre-soaking or wet diets are preferred. Advantageously, the polypeptides of the invention may continue to degrade cellulose in the feed *in vivo.* Fungal based polypeptides of the invention in particular generally have lower pH optima and are capable of releasing important nutrients in such acidic environments as the stomach of an animal. The invention thus also contemplates (e.g. animal) feeds or foodstuffs comprising one or more polypeptides of the invention.

The polypeptides of the invention may also be used during the production of milk substitutes (or replacers) from soy bean. These milk substitutes can be consumed by both humans and animals. A typical problem during the preparation of these milk substitutes is the high viscosity of the soy bean slurry, resulting in the need for an undesirable dilution of the slurry to a concentration of dry solids of 10 to 15%. An enzyme preparation containing a polypeptide of the invention can be added to, or during the processing of, the slurry, enabling processing at a higher concentration (typically 40 to 50%) dry solids. The enzyme may also be used in the preparation of savoury product(s), e.g. from soy bean.

The composition may additionally comprise (particularly when being formulated for use in animal feed) one or more ionophores, oxidising agents, surfactants, rumen protected amino acids, enzyme enhancers or enzymes which may be produced naturally in the gastro-intestinal tract of the animals to be fed.

When added to feeds (including silage) for ruminants or monogastric animals (eg. poultry or swine) the feeds may comprise cereals such as barley, wheat, maize, rye or oats or cereal by-products such as wheat bran or maize bran, or other plant materials such as soy beans and other legumes. The enzyme(s) may significantly improve the break-down of plant cell walls which leads to better utilisation of the plant nutrients by the animal. As a consequence, growth rate and/or feed conversion may be improved. The polypeptides of the invention may be added to the feed (directly or as an additive or ingredient) or treated cellulose (e.g.xylan) may be added instead.

The protein may reduce the viscosity of the feed (containing xylan): the protein may continue to hydrolyse xylan *in vivo.* The proteins of the invention are particularly applicable to animal feeds as they may still be active under highly acidic conditions, such as in the stomach of animals.

A particularly preferred method for the (exogenous) addition of the modified xylanase is to add the polypeptide of the invention as transgenic plant material and/or (e.g. transgenic) seed. The polypeptide may thus have been synthesized through heterologous gene expression, for example the gene encoding the desired enzyme may be cloned in to a plant expression vector, under control of the appropriate plant expression signals, e.g. a tissue specific promoter, such as a seed specific promoter. The expression vector containing the gene encoding the polypeptide can be subsequently transformed into plant cells, and transformed cells can be selected for regeneration into whole plants. The thus obtained transgenic plants can be grown and harvested, and those parts of the plants containing the heterologous (to the plant) polypeptide can be included in one of the compositions, either as such or after further processing. General methods for the (heterologous) expression of enzymes in (transgenic) plants, including methods for seed-specific expression of enzymes, are known⁴⁰. The heterologous polypeptide may be contained in the seed of the transgenic plants or it may be contained in other plant parts such as roots, stems, leaves, wood, flowers, bark and/or fruit. The plant may be a monocot or a dicot. Suitable plants include cereals, such as oats, barley, wheat, maize and rice. Preferably the polynucleotide of the invention is stably incorporated into the plant genome.

The addition of the polypeptide in the form of transgenic plant material, e.g. in transgenic seed may require the processing of the plant material so as to make the enzyme available, or at least improve its availability. Such processing techniques may include various mechanical (eg. milling and/or grinding) techniques or thermomechanical treatments such as extrusion or expansion.

The present invention thus also relates to a process for promoting growth and/or feed conversion in a monogastric or non-ruminant animal, the process comprising feeding the animal polypeptide of the invention. Suitable animals include farm, monogastric and/or non-ruminant animals such as pigs (or piglets), poultry (such as chickens, turkeys), calves or veal or aquatic (e.g. marine) animals (for example fish).

### Assays for Cellulose Degrading Enzymes

Also within the present invention is the use of polypeptides according to the invention in screening methods to identify compounds that may act as agonists or antagonists which may modulate the xylanase. In general terms, such screening methods may involve contacting a polypeptide of the invention with a test compound and then measuring activity or incubating a polypeptide of the invention with a test substance and then detecting any modulation of xylanase activity. Agents which bind to the polypeptides of the present invention can also be identified by binding assays.

Modulator activity can be determined by contacting cells expressing a polypeptide of the invention with a substance under investigation and by monitoring the effect mediated by the polypeptides. The cells expressing the polypeptide may be *in vitro* and preferably, the assay is carried out *in vitro* using cells expressing recombinant polypeptide.

The assays and substrates described herein have allowed identification and confirmation of xylanase activity. However, these assays can be used to detect other cellulose degrading enzymes, whether or not they have xylanase activity. The substrate that can be used for this assay can comprise xylan.

Another aspect of the invention relates to an assay for identifying or detecting a polypeptide which is able to degrade cellulose. The activity may be a xylanase, or, may be pectin lyase, polygalacturonase, esterase, cellulase, xyloglucanase, galactonase, arabinanase or rahamnogalacturonase. The assay may comprise:
(a) providing, as a substrate for a candidate compound (usually a polypeptide) the substrate described in the previous paragraph; and
(b) contacting the substrate with the candidate compound, and detecting whether any products of xylanase activity are produced.

The above assays can be employed to identify modulators of the xylanase activity. Such compounds may reduce the softening of fruit, which may allow better flavour and colour development of fruit and may also allow for longer shelf and/or shipping life. Thus these assays may be used to identify inhibitors of the polypeptides of the invention that may be able to inhibit fruit softening.

Preferred features and characteristics of one aspect of the invention are applicable to another aspect *mutatis mutandis.*

The invention will now be described with reference to the following Examples which are intended to be illustrative only and not limiting.

### EXAMPLES

The aim was to create new xylanases that could function under acidic conditions as demanded by industry, and in particular showed improved thermostability. Many industrial applications of xylanases (such as bleaching pulp and in baking) are conducted at higher temperatures, and it is therefore advantageous to be able to use xylanases that are still active at elevated temperatures. However, increased thermostability is not desireable if it is at the expense of reducing the ability of the enzyme to act under acidic conditions. The aim was therefore to look at mutations that did not cause a shift of the pH optimum towards alkaline conditions (or if there was a shift, it was only slight) and this meant excluding mutations that did not significantly or substantially alter the structure of the enzyme. For this reason inclusion of additional disulphide bridges was rejected.

It was decided to use a fungal xylanase from Family 11 (formerly G-type). The starting enzyme chosen for mutation was the endo-1,4-β-xylanase I (*xlnA I*) from *Aspergillus niger,* strain DS16813 (previously deposited at the Centraal Bureau for Schimmelcultures, Baarn, The Netherlands, under Accession No. CBS 323.90). The amino acid sequence for this enzyme has already been described^{12,13} and the sequence of the *xlynA I gene* has also been disclosed¹². Note that *A. niger* strain DS16813 has been reclassified as *A. tubingensis*¹².

The polypeptide derived from the gene is 211 amino acids in length. A 17 residue long hydrophobic signal sequence is found at the N-terminus of the polypeptide, followed by a pro-peptide which is 12 residues long. The mature protein is 184 amino acids in length with a predicted molecular weight of 19kD. The purified enzyme has a MW of about 25kD. It has a particularly low pH optimum (about 3.0). This is unusual since most G-type enzymes have a pH optimum at around 5.0.

Family 11 xylanases are often characterised by having an aspartic acid residue at position 37 in the mature sequence. This is true of the highly acidic xylanase chosen for mutation^{11,13}. In a less acidic xylanase from *A. tubingensis*¹⁵ at the corresponding position in the xylanase (residue 43) an asparagine residue is found (see Figure 1). The exact position of these residues in Family 11 can be found via a sequence alignment where the amino acid sequence of the *A. niger* endoxylanase is aligned with the sequences of the other Family 11 xylanases (this is shown in Figure 1). If the 3-D structure is known then the position can be found by superposition of the 3-D structures instead.

The 3-D structure of the endoxylanase mutated in the invention has been determined¹¹ and deposited as IUKR (this can be retrieved from SWISS-PROT, Accession Code P55331, XYN1ASPTU:EMBL L2698 ATXLNA1). This sequence differs in only one position from the published muture sequence¹³.

Although Family 11 fungal xylanases have advantages for certain applications, in particular those that require higher activity at acidic pH, the low resistance to elevated temperatures is a major drawback of these xylanases. This sensitivity to temperature can adversly effect yields during production and subsequent handling of the enyzme. In addition, exposure to elevated temperatures during storage can lead to inactivation.

When using xylanases as a feed additive, the xylanase should desirably resist high temperatures that occur, for example during the pelletting process. This can be partially overcome by overdosing the xylanase, in other words simply by increasing the concentration. However, this is expensive and wasteful. Therefore, Family 11 fungal xylanases with improved thermostability are desirable, and would allow for a more efficient use of these xylanases in existing applications and may open up new applications where, until now, stability has been the limiting factor.

Two approaches may be followed. The first is to screen naturally occuring xylanases to find ones with the desired property. The second is to use protein engineering techniques to improve the properties of existing xylanase. In the present invention the latter approach was taken.

Wild-type thermostable Family 11 xylanase have been reported, but these are not of fungal origin, and therefore do not have the desired low pH optimum. In addition, these have a much higher molecular weight. It is also often difficult to obtain enough enzyme for testing.

By contrast protein engineering allows changes to be made at certain positions in the protein which might improved desired properties such thermostability, while maintaining other useful properties (such as activity at low pH) which may be crucial in production handling performance. It also means that an established production process can be modified relatively easily to make the mutant, for example by using a different organism.

In general the average protein offers an almost endless number of possibilities for modifications of the amino acid sequence. Substitution at various positions are the most common. A general rule is that substitution should increase the number and strength of favourable interactions within the protein and should decrease the number and strength of unfavourable interactions. This is of course only possible if the three dimensional structure is available. Whether a potentially stabilising substitution will actually improve stability additionally depends on the particular environment of the amino acid residue being substituted. Although in theory stabilising substitutions can lead to an improvement in overall stability, in fact this depends strongly on the environmental conditions of that residue. Indirect interactions, which are often more subtle and are not so easily mapped from a 3D structure, are more difficult to decipher and to predict the result of changes. Mutations that are shown to stabilise one protein may not show any effect, or may even show an adverse effect, in a homologue. Only in cases of high sequence homology where the local environment is nearly identical can there be a transfer of stabilising mutation from one protein being applicable to another.

Due to the constraints of the environmental context of an amino acid it is very difficult to predict when the general rules for stabilising a protein will hold for a certain substitution. Therefore a random mutagenesis approach was chosen as the method to improve the thermostability of xylanase. The most common approaches such as error prone PCR, spiked oligo's, chemicals, mutator strains etc have certain disadvantages. It is not known where the mutations occur. Bias towards accumulating mutations in certain region has to be avoided. In order to include as many of the potential point mutations as possible the redundancy of the library should be quite high, which requires testing large number of variants. The randomness of the process also implies that mutations will be spread over the whole protein. As a consequence also other vital amino acids may be substituted disrupting a desired functionality of the enzyme

Therefore we have chosen for a site directed random approach. The advantage of this method is that mutagenesis in regions that are vital for enzymatic functionality can be avoided. As the amino acid position at which mutagenesis is performed is known, this approach immediately identifies the positions, which are essential for a certain desired property, without further sequencing. By targeting mutagenesis to a certain position we can be more confident that the target positions are indeed mutated.

In order to avoid disruption of vital functionalities the 3D structure of *A.tubigensis* (UKR) was searched for a region which would lay as much as possible outside the active site and which was not expected to be involved in interactions with the xylan polymer. In principle a similar exercise can be performed using the 3D structure of other family 11 xylanase. A 3D structural alignment allows for establishing of corresponding residues and for assigning functional properties to these residues. In case only the amino acid sequence is known a fairly rough estimate on structural and functional properties can be derived via alignment of the sequence with 3D structures. Subsequent homology modelling allows for building a 3D model which can be used to establish structure-function relationships.

The selected patch in *A. tubingensis* endoI xylanase includes residue Ser 136 to residue Ser160. This segment starts just after β-strand B7 and comprises a 4-residue bend, the β-strand A6, the α-helix, and the turn connecting the helix with β-strand B8. In addition a second segment was selected for mutagenesis: Asp 88 to Tyr 130 . Apart from β-strands B9 and B8 which are not expected to interact with the xylan polymer, this segment also contains the so-called "cord" and "thumb". Both the "cord" and the "thumb" are likely to interact with the substrate, although both do not comprise essential catalytic residues. Both segments have been shown to be quite flexible. As such they might be a target for stabilisation. As stated above the residues in the indicated segments have been subjected to random mutagenesis one by one. In this stage for every triplet only the first two bases were randomised. As a consequence not all twenty amino acids will be included at each position. As the method has proven to work very smoothly, technically randomisation of the whole triplet seems to be very well feasible. For each position about a hundred clones were grown and the produced xylanase were subjected to a stress (relative thermostability) test.

### MATERIALS

| Abbreviations | |
|---|---|
| kb | kilo base |
| bp | base pair |
| oligo | oligonucleotide |
| o/n | overnight |
| PCR | Polymerase Chain Reaction |
| PDA | Potato Dextrose Agar |

| | |
|---|---|
| w/v | weight / volume |

### Strains and Source

| |
|---|
| *E. coli* JM101hsdsA |
| *K. lactis* SL56Gal2B⁴³ |
| *E. coli* TOP10F Invitrogen (KNM 2030-01) |
| *A. niger* ISO502 |

### Plasmids and Source

| | |
|---|---|
| pCR2.1 | Invitrogen, UK |
| pGBCHY4 | |
| XYL3¹² | |
| TZ18R/N⁴⁴ | |

### Materials

| | |
|---|---|
| Restriction enzymes from BRL | |
| T4-DNA ligase | BRL |
| Amplitaq™ | Perkin Elmer |

### General procedures

Standard molecular cloning techniques such as DNA isolation, gel electrophoresis, blotting, enzymatic restriction modifications and ligations of nucleic acids, Southern analyses, *E. coli* transformation, etc., were performed as known^{16,17}. Plasmid DNA was isolated using the QIAprep 8 Miniprep Kit from Qiagen according to the manufacturer's protocol. Synthetic oligo deoxynucleotides were obtained from ISOGEN Bioscience (Maarssen, The Netherlands). DNA sequence analyses were performed on an Applied Biosystems 373A DNA sequencer, according to supplier's instructions.

DNA labelling and hybridisations were conducted according to the ECL™direct nucleic acid labelling and detection systems (Amersham Life Science, Little Chalfont, England) or according to standard radioactive labelling techniques¹⁶. The different enzymes used (restriction enzymes, polymerases, ligases, etc) were purchased from Gibco BRL and/or Perkin Elmer.

### Purification of PCR fragments

PCR fragments were purified with the QIAquick™ purification kit from QIAGEN (Hilden, Germany) according to manufacturer's instructions.

### PCR

PCR was performed with AmpliTaq™, purchased from Perkin Elmer, according to the manufacturer's protocol: reactions were performed in 50 µl containing 250 pg from each oligo, 10 ng template DNA, 1 X Amplitaq™ buffer, 1 X DVB (1 mM Tris pH 8.0, 1 mM NaCl, 0.1 mM EDTA), 10 µM from each dNTP and 1.3 U Amplitaq™ polymerase (added after the first heating step). The reaction mix (without Amplitaq™ polymerase) was incubated for 5 minutes at 95°C, cooled down to 72°C upon which the Amplitaq™ polymerase was added and a cycle program (25 cycles) started. Each cycle comprised 1 minute at 94°C followed by respectively 1 minute at 55°C and 1 minute 30 seconds at 72°C. Finally, the PCR was terminated with an incubation at 72°C for 7 minutes.

### Transformation of K. lactis

Transformation of *K. lactis* was performed either using a LiCl₂ method¹⁸ or via electroporation. In the electroporation method, 5 - 25 µl of an overnight culture of *K. lactis* SL56Gal2B in YEPD is inoculated in 100 ml YEPD (10 g/l yeast extract, 20 g/l bacto-peptone, 2% (w/v) glucose) and grown overnight at 30°C. Growth was continued until 0.4<OD620 <0.6. This procedure was then continued with a 50 ml culture. Cells were pelleted (5 minutes, 3000 rpm, 4°C), washed at 4°C with 50 ml EB (10 mM Tris/HCl, pH 7.5, 270 mM sucrose, 1 mM MgCl₂), upon which the pellet was resuspended in 50 ml YEPD supplemented with 25 mM DTT and 20 mM HEPES (pH 8.0). After an incubation for 30 minutes at 30°C the cells were again pelleted (5 minutes, 3000 rpm, 4°C), washed with 10 ml cold EB and finally resuspended in 1-2 ml cold EB. To 100 µl of the thus obtained cell suspension 0.5 to 1 µg of DNA (linearised via SacII digestion and subsequently purified via the QIAquick PCR method) was added and electroporation was performed at 500 V, 800 and 25 µF. Immediately after the electro-shock 1 ml fresh YEPD medium was added followed by incubation at 30°C, 100 rpm for 2-3 hours. Finally, the cells were collected via gentle centrifugation and spread on selective agar-containing plate media and incubated for 3 days at 30°C. For each transformation 15 µg of plasmid DNA was digested with SstII, purified via chloroform extraction and alcohol precipitated.

### Isolation of K. lactis chromosomal DNA

Chromosomal DNA was isolated using the 'nucleon MiY for yeast DNA extraction' kit from Amersham. The method was adapted for the use of 4.5 ml cultures and after the addition of solution B (see manufacturer's manual) an additional 20 minutes incubation step at 70°C was introduced.

### Expressing xylanase mutants in K. lactis in microtitre plates (MTP)

Growth conditions in MTP for *K.lactis* were as follows. Growth results were obtained using a tomtec incubator. The *K.lactis* EndoI (xyl I) clones were grown in minimal medium 2% glucose + G418 for 4 days at 28°C . In YEPD, an OD value of 2 to 3 fold higher was obtained at the end of the xylanase test (OD about 0.6- 0.7), which allowed measurement accurately to 70 to 80% inactivation. A 70 to 80% inactivation corresponds to an OD of 0.18 to 0.2.

### Xylanase activity assays (the "stress" tests)

Supernatant (pre-diluted when necessary) was diluted 5 times in 0.25 M sodium acetate buffer, pH 4.5. 20 µl of diluted supernatant was transferred to microtiter dishes and 50 µl substrate (4% w/v Remazol Brilliant Blues RBB-Xylan, Megazyme, 2/11 Ponderosa Parade, Warriewood NSW 2101, Australia) dissolved at 70°C in demineralized water) was added and mixed thoroughly by pipetting up and down. The reaction was incubated for 30 minutes at room temperature. The reaction was stopped by addition of 200 µl 96% ethanol and incubated for 10 minutes at room temperature. After the reaction has been terminated the microtiter plates were centrifuged for 10 minutes at 2500 rpm in a Beckman™ GPK centrifuge at room temperature. 100 µl of the supernatant was transferred to a new microtiter dish and absorbance of the blue colour was measured spectrophotometrically at 620 nm in an Anthosreader (Proton and Wilton). Specific activity was calculated from a calibration curve using a xylanase standard dissolved in 0.25 M sodium acetate buffer pH 4.5. The measurements are indicative of xylanase activity in the range of 0-150 EXU/ml. One EXU is defined as the amount of enzyme that liberates 4.53 µmol reducing sugars (measured as xylose equivalents) per minute under assay conditions. Assay conditions comprise: 5mg/ml arabinoxylan from wheat flour (Megazyme) in 100mM sodium citrate buffer (pH=3.5), temperature 40°C, reaction time 60 minutes. Reactions were stopped by adding 1M NaOH. Detection was done colorimetrically at 420nm after incubating the samples with Fe-III-hexacyanide.

In addition to the above absolute determination of xylanase activity a relative method was used that followed the decrease in viscosity of a solution of wheat arabinoxylan (Megazyme, 2/11 Ponderosa Parade, Warriewood NSW 2101, Australia) upon addition of a certain amount of enzyme. Wheat arabinoxylan was dissolved in 0.425M sodium citrate buffer pH 3.5 to a concentration of 8.3 mg/ml. The substrate was incubated at 55°C for 10 minutes. Subsequently a small amount of enzyme (in the range 0.01-0.05 Units/ml) was added and the reaction allowed to proceed. At 60 minutes reaction time the viscosity of the sample was determined relative to a reference sample which was incubated with a standard endoxylanase of known enzymatic activity. Absolute activities for the standard were determined by reducing suger method using Fe-III-hexacyanide.

Enzyme activity according to the XPU definition was measured by detecting reducing sugars using 4-hydroxybenzoic acid hydrazide (PAHBAH). One XPU of activity is defined as the amount of enzyme required to release one µmol reducing sugars produced per minute from wheat arabinoxylan at pH 5.0 and 60°C during 15 minutes, using a calibration curve of D(+)xylose. The assay was performed¹⁹ but with some modifications. The modification is to the PAHBAH reagent as follows : 0.05 M trisodium citrate, 0.1 M Na₂SO₃, 0.02M CaCl₂, 0.5M NaOH and 0.1M p-hydroxybenzoicacid hydrazide (PAHBAH). Final pH was 12. The reagent contain PAHBAH in alkaline solution, stored at room temperature, and was used within one day. The absorbance was measured at 420 nm. A blank was prepared by adding 100 µl 0.1M sodiumacetate buffer instead of enzyme solution. Xylanase activity was assayed by mixing 100 µl of (diluted) enzyme solution with 400 µl 0.35 % wheat arabinoxylan (Megazyme) in 0.1 M sodium acetate buffer pH 5.0. The Eppendorf cups with the substrate are pre-incubated for 5 minutes at 60°C. The reaction is started by adding the enzyme solution. After 15 minutes adding 1.0 ml PAHBAH-reagent terminates the reaction. The Eppendorf cups were heated during 5 minutes at 100°C and then cooled on ice. Samples were centrifuged at the appropriate speed in order to spin down any solid materials eg 1 minute at full speed in a Beckman Microfuge E. The absorbance was measured at 420 nm. A blank is prepared by adding 100 µl instead of enzyme solution. The measuring range was 0.01-0.1 PAHBAH-U/ml.

### Purification of the Xylanases

Mycelium was removed by centrifugation. The filtrates were concentrated by ultrafiltration and transferred to 10mM sodiumacetate buffer, pH=4.1. The sample was applied to a resource Q™ ion exchange column (Pharmacia) and eluted in 20 column volumes starting with buffer A (10mM sodium acetate) to 35% buffer B (10mM sodium acetate containing 1 M NaCl) with a flow of 4ml/min. The most active fraction was applied to a Sephacryl™ S200 column (HiPrep 16/60, High Resolution, Pharmacia) and eluted at 1ml/min using 0.25M sodium acetate (pH 4.5). Purity was determined with SDS-PAGE and analytical HPLC size exclusion chromatography .

### Summary of Screening Protocol of the Mutants

| Step | Protocol Stability Testing Clones |
|---|---|
| Setting up the plates | Mutant clones were distributed randomly over 96 wells microtiter plates (MTP). In every plate about 10-20 wild type clones were included randomly as a control : plate MTP_I |
| Production of the Enzyme | In MTP_I: 220µl Yeast Minimal Medium 2% glucose + 50 µg/ml G418 for 4 days at 28°C |
| Removal of Cells | Centrifugation at 3000rpm for 15 minutes |
| Sample Preparation and Handling before Stress Test | Transfer to MTP_II: Take 100 µl of supernatant from MTP_I and add 50 µl of 0.25 M sodium acetate buffer (pH=4.5): sample A. Enzyme concentration was then in the range 5-40 EXU/ml |
| Stress Test Dosing | Transfer to MTP_III plate for stress test: 100 µl sample A |
| Controls Dosing | MTP_II maintained as control: 50 µl sample A |
| Stress Test Sample Handling | Incubate MTP_III in a PCR cycler/incubator or water bath at 51°C for 27 minutes and 30 seconds |
| Handling Control | Leave MTP_II control plate at room temperature |
| Sample Handling after Stress Test | MTP_III is left for approx 10-15 minutes after heating at room temperature to cool down. |
| Determination of the Residual Activity | Remazol Brilliant Blue RBB-xylan activity: Add 50µl 4.5 % RBB Xylan in 80mM NaAc/Hac buffer (pH 4.5) to 50µl MTP_III heated supernatant and to 50µl MTP_II control supernatant and incubated for 30 minutes at 30°C. Subsequently 250µl of 96% ethanol was added. After shaking for 5 minutes, it was centrifuged for 15 minutes at 3000rpm at 4°C. Transferred 100µl of the supernatant to new MTP plates MTP_IV heated and MTP_IV control and measure OD at 630nm |
| Intepretation Data | Compare activity of the control MTP_IV with residual activity after the stress test MTV_IV: determine ratio OD-heated MTV_IV/OD-control MTV_IV. Compare this ratio with corresponding ratio of the wild type measured in the same plate. |

### Measuring the thermostability of mutant clones grown in shake flasks.

| Step | Protocol Stability Testing Clones expressed in shake flask |
|---|---|
| Producing the Enzyme | Shake Flask : YEP2D medium, 10g/l yeast extract, 20g/l bactopeptone, 4% glucose for 3 days at 30°C, 280rpm |
| Removal of Cells | Centrifugation and sterile filtration |
| Sample Preparation and Handling before Stress Test | Gelfiltration on PD10 in order to transfer the sample to 0.25M sodium acetate buffer (pH 4.5). |
| Enzyme Concentration | Range 10-100 EXU/ml |
| Stress Test Dosing | 500 µl or 1000 µl |
| Controls Dosing | 500 µl or 1000 µl |
| Stress Test Sample Handling | Incubate Eppendorf tubes in thermomixer at 51°C (after 2-3 minutes at sample temperature of 51°C). |
| Handling Control | The control is not put in the thermomixer but transferred at t=0 to ice and stored in the same way as stressed samples |
| Sample Handling after Stress Test | Eppendorf tubes were withdrawn from the thermomixer at 30,60,120 minutes and put into ice immediately. The samples are left in ice for 1-2 hours before they are assayed |
| Determination of the Residual Activity | Residual Activity was determined via viscosimetric assay. Details : substrate 0.7% arabinoxylane (Megazyme) in 0.1M citric acid buffer pH 3.5. Incubation in the viscorobot during 30 minutes at 40°C followed by viscosity measurement |
| Intepretation Data | Activity versus incubation time was plotted for mutant as well as wild type. |

### Expression of xylanase in K.lactis

Initially it was planned to make a xylanase-mutant expression library in *E.coli* and screen this library for more thermostable mutants. As already described xylanases can not be expressed in *E.coli* and therefore another host organism had to be selected. So it was decided to investigate if *K.lactis* is a suitable host for the expression of xylanase (XlnA). This means that the XlnA production with only one copy of the integrated expression-vector was high enough for xylanase activity to be measured in the available screening method (microtitreplate assay previously described).

To test this, a xylanase expression vector based on pGBCHY4 was made and transformed to *K.lactis* SL56Gal2b. This vector contains the *K. lactis* Lac4 promoter to drive high expression in *K. lactis*, the *K. lactis* Lac4 terminator, and the *K.lactis* alpha factor signal sequence was used for secretion. The selection marker was G418.

### Construction of pGBTXY14 (bkj96002)

To obtain an intron free XlnA gene a fusion PCR with pXYL3 as a template was performed. To make a correct connection between the lac promoter and the mature xylanase gene an NdeI site was introduced just in front of the first amino acid codon.

The 5'part of the mature XlnA was generated via PCR with oligo AB6855 and AB 6856, located just in front of the mature protein which introduced the required NdeI site. The 3'part of the gene was isolated with oligo AB 6857 and AB 6858, the latter containing a HindIII site after the stop codon.

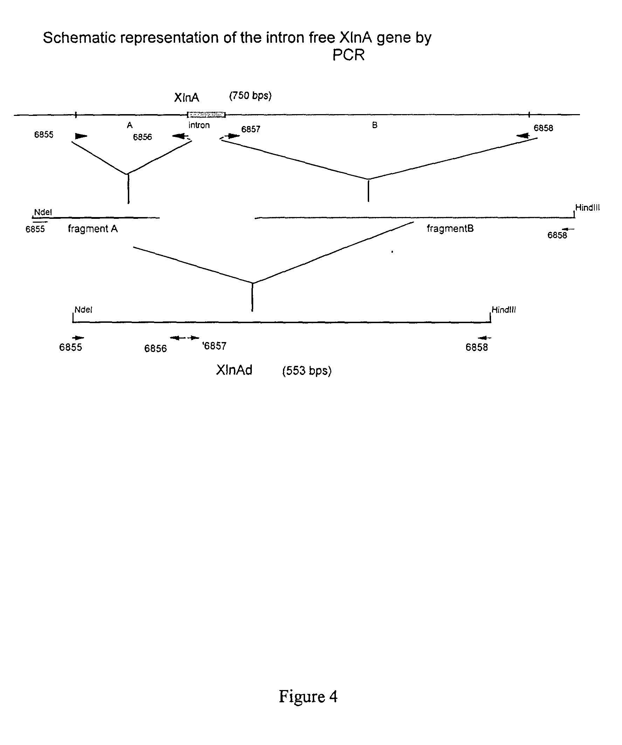

To isolate the full length mature XlnA a second PCR was performed using two fragments described previously as templates and with the oligos AB6855 and 6858. A schematic representation of the fusion PCR is shown in Figure 4.

The obtained fragment was digested with NdeI and HindIII and ligated into pTZ18R/N similarly digested with NdeI and HindIII, the ligation was transformed to CaCl₂ competent *E. coli* JM101hsdsA. Transformants were selected on LC+ containing ampicillin as a selection marker and IPTG and X-Gal for blue/white screening.

From 3 white colonies plasmid DNA was isolated and first checked by restriction enzyme analysis for the correct insert. All transformants had an insert of the correct length. One of these transformants was then checked by sequence analysis and had the expected sequence. This plasmid was called pGBTXY14. Figure 4 shows the schematic representation of the intron free *XlnA* gene by PCR.

### Construction of the expression-vector pGBTXY6

To obtain a *K.lactis* xylanase expression vector the chymosin gene in pGBCHY4 was replaced by the XlnA gene obtained from pGBTXY14. First a correct fusion between the *K.lactis* α factor-leader and mature XlnA was made by fusion PCR (using PCR as described above). The mature part of the XlnA gene was isolated by PCR with oligos AB7096 and AB7097 with pGBTXY14 as a template and the *K.lactis* α factor leader was isolated with oligos AB7098 and AB7099 from pGBChy4 followed by a fusion PCR from the two PCR products and oligos AB7097 and AB7098. This fusion product was cloned into the TA cloning-vector pCR2.1 (Invitrogen) resulting in pGBTXY16.

The final expression vector was made by digesting pGBChy4 with BssHII and XhoI and replacing the insert with the pGBTXY16 BssHII/XhoI derived fragment resulting in the *K lactis* xylanase expression-vector pGBTXY6. The insert was checked by sequence analysis.

### Xylanase production by K. lactis transformants

According to a standard protocol 15 µg pGBTXY6 was digested with SacII to liberate the pTZ18R moiety (necessary for stable replication in *E. coli)* from the expression cassette. The DNA was purified via a chloroform extraction followed by an alcohol precipitation, dissolved in 15 µl TE and transformed to *K.lactis* SL56Gal2B using the LiCl₂-method. After 2 days colonies could be picked: 8 large and 8 small colonies were selected randomly. After purification the 16 isolated transformant, were first tested for xylanase production. Transformants were grown in YEP2D during 3 days at 30°C and 300 rpm, upon which 10 ml culture was centrifuged (pellets were stored at -20°C) and xylanase production was measured in the supematants using a microtiter-plate assay with 4% RBB-xylan as a substrate. Xylanase production varied from 10 to 220 EXU/ml. No production differences were observed between transformants derived from the small and large colonies.

By immunoblotting the amount of protein was estimated and at the same time quality control (with respect to correct processing and degradation of the produced xylanase) was established.

For the immunoblot 20 µl of culture supernatant was mixed with 5 µl sample-buffer, 1 µl β-mercaptoethanol and 1 µl bromophenol blue. The mixture was boiled for 3 minutes, centrifuged and 13.5 µl layered on an SDS-PAGE. As a positive control the *A. niger* EndoI xylanase was used, and as a negative control supernatant from the 'empty' (containing no xylanase expression cassette) host strain *K.lactis* S156Gal 2B.

In all transformants a xylanase protein of the correct size could be detected. From these experiments it was concluded that all the isolated transformants did produce xylanase.

To compare the xylanase production levels of constructs containing the (endogenous) *A. niger* xylanase signal sequence with constructs containing the *K.lactis* alpha factor signal sequence, pGBHSA20xyl (a construct similar to pGBTXY6 but containing the xylanase signal sequence) was transformed into *K.lactis* SL56Gal2B. In parallel pGBTXY6 was transformed (again) into this strain.With both constructs normal transformation frequencies were obtained. Ten transformants from pGBTXY6 and six from pGBHSA20xyl were first purified and then tested for their xylanase production as described above. Two series of shake flask experiments were performed. In the first series the production levels from the pGBTXY6 transformants varied from 40 to 170 EXU/ml, while in only two of the pGBHSA20xyl transformants xylanase activity could be measured (30 and 60 EXU/ml, respectively).

In the second series the production levels from the pGBTXY6 transformants varied from 70 to 260 EXU/ml and the expression levels from the pGBHSA20xyl transformants varied from 40 to 270 EXU/ml. From each construct 7 transformants were also analyzed by immunoblotting.

In all the pGBTXY6 transformants a main band at the position of the xylanase control was found. The intensity of this band correlated with the measured activities. In the supernatant of the pGBHSA20xyl transformants two bands in an almost 1:1 ratio were present. The first band was at the position of the mature xylanase, the second band was slightly larger (xylanase + signal sequence) and was most likely a pre-xylanase (indicating that only part of the produced xylanase was correctly processed). This could also explain the lower xylanase activities found in the transformants with this construct. From these results it was concluded that pGBTXY6 expression vector should be used for XlnA production in *K. lactis.*

### Copy number determination of K.lactis transformants

This was to establish the correlation between the copy number (no. of copies of the gene in the organism) and the xylanase production and to make sure that the screening assay could readily detect the production of a one-copy xylanase *K. lactis* strain in a screening format. From 3 transformants, designated pklaxyl 1, 6 and 11, the copy-number was determined.

Chromosomal DNAs of these transformants and from the host strain was digested with *Hin*dIII, seperated on a 0.7 % agarose gel, blotted and hybridized with the LAC4 promoter probe.

From the hybridization results (not shown) it is clear that pklaxyl 1 had only integrated one copy of the expression-vector and pklaxyl 11 had two copies tandem integrated in one of the LAC4 loci. The situation in pklaxyl 6 was not clear it seems that the expression vector was not integrated in the LAC4 promotor, but the estimated copy-number was 5 to 6. (The EXU value in brackets shows the variation between transformants).

**Table 1:**

| Xylanase production in *K.lactis* SL56GAL2B transformants | | |
|---|---|---|
| transformant number | EXU/ml | copy number |
| 1 | 80 (50) | 1 |
| 6 | 250 (150) | 5/6 |
| 11 | 140 | 2 |

### Random site directed mutagenesis: strategy

This is based on the site-directed mutagenisis by PCR but here PCR was performed with a degenerate oligo. The degenerate oligo is designed in such away that only one amino acid position is mutated (only the triplet coding for the amino acid to be changed was mutated). In this example only the first and the second nucleotides of the triplet coding for a specific amino acid were changed (into NN) thus ensuring that that particular position was changed into a subset of all 20 amino acids. Which amino acid and the number of different amino acids that can be introduced at a specific position depends on the last nucleotide. Only if the whole triplet is degenerate (NNN) can all amino acids can be obtained at one particular position.

**Table 2:**

| This shows the missing amino acid if only the first two nucleotides of a codon are changed. In the first row the last nucleotide of a triplet is indicated. The column under the nucleotide indicates the amino acids that will be missing (unobtainable) if the third nucleotide is kept constant. | | | |
|---|---|---|---|
| T | A | G | C |
| | | | |
| Met | Asn | Asn | Gln |
| Lys | Asp | Asp | Glu |
| Glu | Cys | Cys | Lys |
| Gln | His | His | Met |
| Trp | Met | Ile | Trp |
| | Phe | Phe | |
| | Trp | Tyr | |
| | Tyr | | |

**Table 3:**

| This shows the amino acids that can be obtained if the third nucleotide ends at the nucleotide indicated at the top of each of the four columns. | | | |
|---|---|---|---|
| T | A | G | C |
| | | | |
| Ala | Ala | Ala | Ala |
| Arg | Arg | Arg | Arg |
| Asn | Gln | Gln | Asn |
| Asp | Glu | Glu | Asp |
| Cys | Gly | Gly | Cys |
| Gly | Ile | Leu | Gly |
| His | Leu | Lys | His |
| Ile | Lys | Met | Ile |
| Leu | Pro | Pro | Leu |
| Phe | Ser | Ser | Phe |
| Pro | Thr | Thr | Pro |
| Ser | Val | Trp | Ser |
| Thr | | Val | Thr |
| Tyr | | | Tyr |
| Val | | | Val |

It will thus be seen that adding the same nucleotide (column) of Tables 2 and 3 together will give, each time, the full set of 20 amino acids.

In a first round only the first 2 nucleotides of each triplet within two selected regions (amino acid positions 88-125 and positions136 -161) of the XlnA gene were mutated.

### Random site directed mutagenesis protocol

As a template for PCR, plasmid pGBTXY6 was used. A suitable fragment for cloning was obtained via fusion PCR.

For each amino acid position to be changed one degenerate oligo (the 5'PCR oligo) was used in combination with the 3'oligo olxyl2 (AB9434). The resulting PCR fragment 1 had random mutations at the target amino acid position.

The second fragment was obtained with the 5'oligo olxyl1 (AB9433). The 3'oligo was situated at the 5'site of the mutated position (not overlapping the mutation) with an overlap of 21 nucleotides with PCR fragment 1.

Next a fusion PCR was performed with PCR fragments 1 and 2 and oligos olxyl1 and olxyl2.

The obtained PCR fragments were digested with SstI and XhoI resulting in two fragments. One fragment was isolated and ligated into SstI/XhoI fragment of the expression vector pGBTXY6.

The ligations were transformed to competent *E.coli* (JM101hsds 649) cells. Transformants were grown for 3 days at room temperature. All transformants obtained for one amino acid position were pooled after resuspension from plates in 2 x TY/50 µg/ml ampicillin. Half of the cell suspension was stored as a glycerol culture. The rest of the cell-suspension was used for the isolation of plasmid DNA. The isolated plasmid DNAs were digested with SstI and XhoI. All isolates had an insert of the correct length.

To check the mutation frequency obtained with this method independent transformants were sequenced.

From each mutated amino acid position approximately 100 transformants (in total for all positions approximately 7000 transformants) were tested for their thermostability.

### COMPARATIVE EXAMPLE 2

### Insertion of a Disulphide Bridge

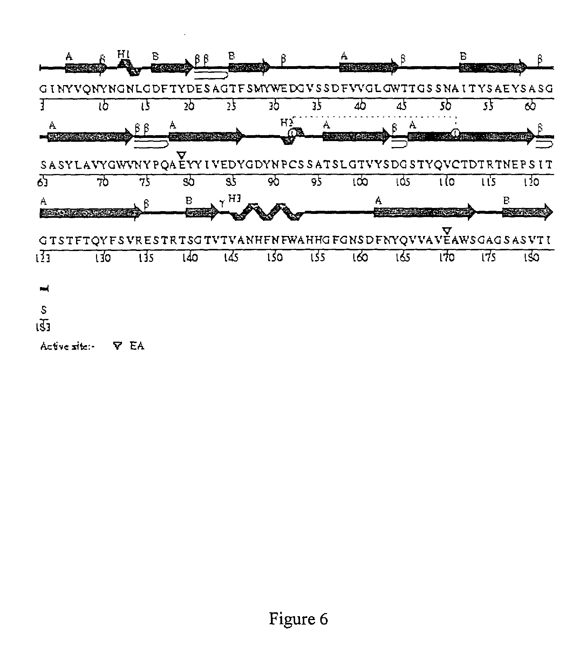

In order to test the effect on thermostability of the enzyme by inserting a disulfide bridge two mutations were made : Ser103Cys and Asn147Cys. The 3D structure UKR_A (see Figure 6) of *A. tubingensis* EndoI suggests that formation of a disulphide bond should be possible when both serine 103 and asparagine 147 residues are replaced by cysteines. At corresponding positions (S100→N148) a disulphide bond has been engineered into Family 11 xylanase of *B. circulans*⁴ and the resulting mutant was claimed to exhibit improved thermostability. This Example was to test whether the same disulphide bridge when introduced into a fungal xylanase, might also have increased thermostability.

In addition alanine at position 146 was replaced by a serine as well as asparagine residue. This was carried out because of the residue at position 146 is in a favourable position for "N-terminal capping" of the only alpha-helix which is found in the EndoI xylanase. However alanine is not a capping residue. N-capping of alpha helices has been shown to increase the thermostability of proteins. The alanine is exposed to solvent and in other Family 11 xylanases more polar residues are found in this position amongst which residues such as serine, threonine, glutamine, asparagine, which are suitable residues for capping at the N-terminal of an alpha helix.

The EndoI mutants were tested for thermostability against the wild type at 50, 55 and 60°C (incubation for 20 minutes at pH 4.5).

**Table 4:**

| Results of thermostability tests | | | |
|---|---|---|---|
| Xylanase | 50° | 55° | 60° (20 minutes) |
| Wild-type | 105 | 59 | 0 |
| Double-Cys (S103C→B147C) | 91 | 55 | 0 |
| A146S | 94 | 52 | 0 |
| A146N | 90 | 42 | 0 |

None of the mutants showed improved thermostability. Although the substitutions in (*B.circulans*) are the same (S100C and N148C) the direct environment of the disulphide bond seems to be substantially different with respect to amino acid composition. So a disulphide bond in a different enzyme and environment did not show the same thermostability effect.

Also the context in which Ala 146 was replaced for more favourable residues (according to prior art suggestions with respect to engineering the capping of helices) did not result in a net increase of favourable interactions leading to increased thermostability.

### EXAMPLE 3

### Analysis of modified xylanses

The mutant library was grown in microtiter plates (MTP). After growth cells were removed by centrifugation. Two samples were taken from the supernatant. One sample was incubated at elevated temperatures, the other stored at room temperature. After the stress test the residual activity of the heated sample (h) was determined and compared to the activity of the non-heated sample (Nh). The same was done for the wild type (wt) enzyme under conditions where the wild-type only showed a residual activity of 10-20% . The selection of more thermostable mutants was based on the ratio (h/Nh)mut: (h/Nh)wt. Where the ratio is larger than one then the mutant shows a higher residual activity than the wild-type. In addition all the wells showing an OD<0.03 after blank correction were excluded. An additional criteria was placed on the blank-corrected OD for non-heated samples: mutants in the OD range 0.03 to 0.09 were omitted because of a too low standard activity. Based on these criteria 444 mutant clones were picked and further tested in duplicate and triplicate. The mutants which showed improvement factors (ratios) of 2 or higher are shown in Tables 5 and 6 (the results for those mutants with increased thermostability ratios of from 1.0 to under 2.0 are not shown).

The type of residue that was substituted and its corresponding position (nr) in the mature wild type *A. niger* xylanase sequence are given. In addition any secondary or other structural features are indicated under 3D. The solvent access or exposure (Acc) of the whole residue (side chain atoms and main chain atoms) is given in Ångstroms²⁰. The variability (Var) of residues indicates the natural substitution frequency on a scale of 0-100, using 70 known xylanase sequences which were present in EMBL/SWISSPROT RELEASE 38.0⁴². This gives an indication of how highly conserved a residue is between different xylanases. In the two right hand columns the coding sequence (codon) is indicated and all possible substituting amino acids at that position when the first two bases are varied randomly.

**Table 5 :**

| Results of stress (activity) tests of xylanase mutants at positions 91-126 | | | | | | | | |
|---|---|---|---|---|---|---|---|---|
| Residue | nr | 3D | Acc | Var | clone no. | Ratio: (h/Nh)mut/ (h/Nh)wt | Codon | Substituting Amino Acid (Codon: XXN) |
| pro | 91 | Helical turn | 4 | 6 | f5 | 4.5 | cct | PGALVIFYSCTNHRD |
| | | | | | f6 | 2.5 | | |
| | | | | | f10 | 2.4 | | |
| | | | | | h3 | 4 | | |
| cys | 92 | Helical turn: disulphide bridge to Cys 111 | 19 | 37 | b9 | 2 | tgc | PGALVIFYSCTNHRD |
| | | | | | f12 (xtm6) | 5.1 | | |
| | | | | | a4 (xtm8) | 3 | | |
| | | | | | g12 (xtm9) | 2.7 | | |
| | | | | | g1(xtm10) | 1.9 | | |
| | | | | | g7 | 2.1 | | |
| ser | 93 | Helical turn | 93 | 33 | d4 | 3 | agt | PGALVIFYSCTNHRD |
| | | | | | d11 | 2.4 | | |
| | | | | | b8 | 3.3 | | |
| | | | | | b4 | 3 | | |
| | | | | | h7 | 6.6 | | |
| | | | | | b11 | 2.0 | | |
| | | | | | a9 | 4.9 | | |
| | | | | | c7 | 3.6 | | |
| | | | | | c9 | 2.4 | | |
| ser 91-93 | | | | | f5 (xtm1) | 4.5 | | |
| ser 91-93 | | | | | g6 (xtm2) | 2.5 | | |
| ser 91-93 | | | | | f10 (xtm3) | 2.4 | | |
| ser 91-93 | | | | | h3 (xtm4) | 4 | | |
| ser | 94 | cord/Bsite secondary | 79 | 36 | h10 | 3.1 | tcg | PGALVMWSTQKRE |
| ser 94-95 | | | | | e8 | 2.9 | | |
| ser 94-95 | | | | | e3 | 3.8 | | |
| ser 94-95 | | | | | b5 | 5.6 | | |
| ala | 95 | cord/Bsite secondary | 17 | 23 | h4 | 4 | gcc | PGALVIFYSCTNHRD |
| ala 94-95 | | | | | h10 | 3.1 | | |
| ala 94-95 | | | | | e8 | 2.9 | | |
| ala 94-95 | | | | | e3 | 3.8 | | |
| ala 94-95 | | | | | b5 | 5.6 | | |
| ser | 97 | b9 | 103 | 54 | g11 | 4 | agc | PGALVIFYSCTNHRD |
| leu | 98 | b9 | 34 | 41 | h5 | 4.5 | ctt | PGALVIFYSCTNHRD |
| | | | | | f5 | 6.6 | | |
| gly | 99 | b9 | 26 | 5 | | | ggt | PGALVIFYSCTNHRD |
| thr | 100 | b9 | 79 | 28 | | | acc | PGALVIFYSCTNHRD |
| val 101-126 | 101 | b9 | 16 | 20 | c2 | 2.0 | gtg | PGALVMWSTQKRE |
| val 101-126 | | | | | d3 | 3.2 | | |
| tyr | 102 | b9 | 153 | 44 | b6 | 2.1 | tac | PGALVIFYSCTNHRD |
| ser | 103 | b9 | 2 | 37 | c5 | 7.3 | tct | PGALVIFYSCTNHRD |
| | | | | | f8 | 6.1 | | |
| | | | | | b8 | 2.6 | | |
| asp | 104 | turn | 41 | 8 | f2 | 3.5 | gat | PGALVIFYSCTNHRD |
| | | | | | f3 | 2 | | |
| ser | 106 | b8 | 6 | 28 | e5 | 1.6 | agc | PGALVIFYSCTNHRD |
| tyr | 108 | b8 | 0 | 3 | h6 | 4.2 | tac | PGALVIFYSCTNHRD |
| | | | | | g11 | 2.2 | | |
| gln | 109 | b8 | 53 | 29 | | | caa | PGALVISTQKRE |
| cys | 111 | b8: disulphide bridge to Cys 92 | 0 | 35 | | | tgc | PGALVIFYSCTNHRD |
| thr 112-113 | 112 | b8 | 21 | 41 | | | acc | PGALVIFYSCTNHRD |
| asp 113-114 | 113 | b8 | 51 | 35 | f10 | 2.0 | gac | PGALVIFYSCTNHRD |
| thr thr 113-114 | 114 | b8 | 81 | 30 | f10 | 2.0 | ac t | PGALVIFYSCTNHRD |
| arg arg 115-116 arg 115-116 | 115 | b8 | 76 | 9 | h2(xtm 11) h6 | 3.3 2.1 | cga | PGALVISTQKRE |
| thr 116-115 | 116 | b8 | 76 | 9 | h2 | 3.3 | cga | PGALVMWSTQKRE |
| asn 117-115 | 117 | b8 | | | h6 | 2.1 | | |
| glu 118-157 | 118 | b8 | 67 | 29 | | | gaa | PGALVMWSTQKRE |
| pro | 119 | b8 | 74 | 7 | | | ccg | PGALVMWSTQKRE |
| ser | 120 | thumb | 19 | 8 | | | tcc | PGALVIFYSCTNHRD |
| ile | 121 | thumb | 50 | 12 | | | atc | PGALVIFYSCTNHRD |
| thr | 122 | thumb | 96 | 38 | e7 d8 b10 c7 a1 | 6.1 2.7 5.5 1.8 2.4 | acg | PGALVMWSTQKRE |
| gly | 123 | thumb | 42 | 2 | | | gga | PGALVISTQKRE |
| thr | 124 | b7 b7 b7 b7 b7 | 104 | 15 | b11 g6 b8(xtm 15) d11 e7(xtm16) | 2.4 3.2 2.0 1.9 2.9 | aca | PGALVISTQKRE |
| ser | 125 | b7 | 21 | 43 | b6 | 2.0 | agc | PGALVIFYSCTNHRD |
| thr 101-126 | 126 | b7 | 75 | 5 | c2 | 2.0 | acg | PGALVMWSTQKRE |
| thr 101-126 | | | | | d3 | 3.2 | | |

**Table 6:**

| Table 6: Results of stress (activity) tested xylanase mutants at positions 136-160 | | | | | | | | |
|---|---|---|---|---|---|---|---|---|
| Residue | nr | 3D | Acc | Var | clone no. | Ratio: | Codon | Substititing Amin Acid (Codon: XXN) |
| | | | | | | (h/Nh)mut/ | | |
| | | | | | | (h/Nh)wt | | |
| ser | 136 | | 80 | 35 | b3 | 4.2 | agc | PGALVIFYSCTNHRD |
| | | | | | b1(xtm21) | 13.6 | | |
| | | | | | f12(xtm22) | 2.1 | | |
| | | | | | a4(xtm25) | 3.1 | | |
| | | | | | b9(xtm24) | 1.8 | | |
| thr | 137 | | 66 | 29 | c10 | 2.1 | acg | PGALVMWSTQKRE |
| | | | | | c12(xtm31) | 2.3 | | |
| | | | | | e10(xtm18) | 1.8 | | |
| | | | | | a3 | 2.3 | | |
| | | | | | a6 | 2.8 | | |
| | | | | | f12 | 2.5 | | |
| | | | | | b4 | 2.4 | | |
| thr 139-140 | 139 | | 45 | 35 | e11 | 2.3 | | |
| ser | 140 | a6 | 61 | 26 | d12 | 3.3 | tct | PGALVIFYSCTNHRD |
| ser 140-139 | | a6 | | | e11(xtm17) | 2.3 | | |
| gly | 141 | a6 | 16 | 4 | | | gga | PGALVISTQKRE |
| thr | 142 | a6 | 48 | 26 | | | acg | PGALVMWSTQKRE |
| val | 143 | a6 | 0 | 8 | | | gtg | PGALVMWSTQKRE |
| thr 144-148 | 144 | a6 | 44 | 31 | | | act | PGALVMWSTQKRE |
| val | 145 | helix | 0 | 36 | f4 | 2.4 | gtt | PGALVIFYSCTNHRD |
| | | capping | | | f5 | 2.2 | | |
| ala | 146 | helix | 25 | 37 | | | gcc | PGALVIFYSCTNHRD |
| asn | 147 | helix | 71 | 31 | | | aac | PGALVIFYSCTNHRD |
| his | 148 | helix | 0 | 2 | | | cat | PGALVIFYSCTNHRD |
| his 148-144 | 148 | helix | 0 | 2 | | | | |
| phe 149-157 | 149 | helix | 0 | 12 | | | ttc | PGALVIFYSCTNHRD |
| asn | 150 | helix | 91 | 29 | | | aac | PGALVIFYSCTNHRD |
| phe | 151 | helix | 35 | 31 | | | ttc | PGALVIFYSCTNHRD |
| trp | 152 | helix | 0 | 0 | | | tgg | PGALVMWSTQKRE |
| ala | 153 | helix | 37 | 32 | g5 | 2.1 | gcg | PGALVMWSTQKRE |
| his | 154 | turn | 142 | 40 | g6(xtm28) | 3.1 | cac | PGALVIFYSCTNHRD |
| his | 155 | turn | 75 | 48 | c8 | 2 | cat | PGALVIFYSCTNHRD |
| | | | | | b11 | 2.7 | | |
| gly | 156 | | 49 | 0 | | | ggg | PGALVMWSTQKRE |
| phe | 157 | | 7 | 14 | | | ttc | PGALVIFYSCTNHRD |
| asn | 159 | | 52 | 36 | g2 | 2.0 | aat | PGALVIFYSCTNHRD |
| ser | 160 | | 66 | 18 | f7 | 2.2 | agc | PGALVIFYSCTNHRD |

A summary of the positions for which amino acid substitution resulted in clones expressing xylanases with improved thermostability (ratio above 2.0) is given in Table 7.

**Table 7:**

| Summary of positions that showed improved thermostability mutants | | | | | | |
|---|---|---|---|---|---|---|
| nr | residue | 3D | Side chain atoms exposed to solvent | | | Substitution frequency |
| | | | CB | OG | CG2 | |
| 92 | cys | disulphide bridge to 111 | Y | n | | 37 |
| 93 | ser | helical turn | Y | y | | 33 |
| 94 | ser 94-95 | Bsite secondary | Y | y | | 36 |
| 95 | ala 94-95 | Bsite secondary | N | | | 23 |
| 97 | ser | b9 | Y | y | | 54 |
| 98 | leu | b9 | CD exposed | | | 41 |
| 101 | val 101-126 | b9 | | | | 20 |
| 102 | tyr | b9 | Completely exposed | | | 44 |
| 103 | ser | b9 | N | y | | 37 |
| 104 | asp | turn | Exposed | | | 8 |
| 106 | ser | b8 | N | y | | 28 |
| 108 | tyr | b8 | Completely buried 3D structure suggestsTyr»Phe | | | 3 |
| 113 | asp 113-114 | b8; Bsite secondary | Solvent exposed | | | 35 |
| 114 | thr 114-113 | b8; | Y | y | y | 30 |
| 115 | arg 115-116 | b8; Bsite secondary | Solvent exposed | | | |
| 116 | thr 116-115 | b8 | Y | y | y | 43 |
| 122 | thr | thumb, turn | Y | y | y | 38 |
| 124 | thr | b7 | Y | y | y | 15 |
| 125 | ser | b7 | Y | n | | 43 |
| 126 | thr 126-101 | b7 | Y | y | y | 5 |
| 136 | ser | | Y | y | | 35 |
| 137 | thr | | N | y | y | 29 |
| 139 | thr 139-140 | | N | y | y | 35 |
| 140 | ser | a6 | Y | y | | 26 |
| 145 | val | helix, capping | Buried | | | 36 |
| 153 | ala | helix | Y | | | 32 |
| 154 | his | turn | Exposed | | | 40 |
| 155 | his | turn | Exposed | | | 48 |
| 159 | asn | | Exposed | | | 36 |
| 160 | ser | | Y | | y | 18 |

(Key: CB = (β-carbon; OG = γ-oxygen; CG2 - γ-carbon)

In a number of cases two positions were mixed since it was not possible in all cases to establish unambigiously the position of mutagenesis. In situations where one of two residues was highly preferred at a position (low variability) then the most variable position was selected. Otherwise both positions were included. For some "doubles" the individual position or at least one of the individual positions were analysed. In case one of the positions did not have an improved clone then it was assumed that this position did not contribute to the observed improvements for that double.

**Table 8:**

| Statistics of Random Site-Directed Mutagenesis | | | | | |
|---|---|---|---|---|---|
| | Residue | No. of this residue in EndoI | No. of Positions having a mutated residue | No. of Positions yielding an improved mutant | score |
| 1 | ser | 28 | 10 | 9 | 90 |
| 2 | thr | 20 | 7 | 7 | 100 |
| 3 | val | 16 | 4 | 2 | 50 |
| 4 | ala | 15 | 3 | 2 | 67 |
| 5 | asp | 9 | 2 | 2 | 100 |
| 6 | asn | 12 | 3 | 1 | 33 |
| 7 | his | 3 | 2 | 2 | 100 |
| 8 | tyr | 17 | 2 | 2 | 100 |
| 9 | leu | 4 | 1 | 1 | 100 |
| 10 | cys | 2 | 2 | 1 | 50 |
| 11 | arg | 3 | 1 | 1 | 100 |
| 12 | gln | 5 | 2 | 0 | 0 |
| 13 | pro | 3 | 2 | 0 | 0 |
| 14 | gly | 19 | 6 | 0 | 0 |
| 15 | ile | 5 | 1 | 0 | 0 |
| 16 | met | 1 | 0 | 0 | 0 |
| 17 | phe | 9 | 4 | 0 | 0 |
| 18 | trp | 5 | 1 | 0 | 0 |
| 19 | glu | 8 | 1 | 0 | 0 |
| 20 | lys | 0 | 0 | 0 | 0 |

Surprisingly it was found that for substitutions at serines, 9 out of the 10 serines that were mutated showed improved resistance to thermal stress. The region which had been subject to mutagenesis encompassed ten serine residues in total (see Table 8). This remarkable result is not yet fully understood. Upon inspecting the 3-dimensional structure the one serine out of the ten that did not show increased thermostability turned out to be the only serine in the region that is completely buried and that is conserved throughout the family 11 xylanases. Extrapolating these results towards serines outside the mutagenesis region this would mean that serines at positions: 22, 27, 48, 49, 55, 59, 61, 173, 177, 179 and 183 are all candidates for mutagenesis in order to improve thermostability. All these serines exhibit an exposed side chain. Serines 63, 65, 132 are completely buried inside the molecule, while the other serines do expose either CB or OG sidechain atoms or both atoms to the solvent.

In case of mixed mutants such as 91-93 for example, mutations may occur at position 91 and 93. In case of 91-93 it is likely that stabilising mutations will occur at position 93, because proline at position 91 is conserved and only moderately accessible through the substrate binding cavity. In addition mutagenesis of only proline did not give positive clones while mutagenesis of position 93 alone gave positive clones. Two clones were sequenced (xm1 and xtm4). This confirmed that mutations were found at position 93 : both showed the mutation S93L (SEQ. ID. No. 4).

In addition it turned out that clone xtm1 produced a mixture of two single point mutants due to the expression host had acquired two different gene copies. The second gene showed the muation A23T. This might explain why the observed stability of clones xtm1 and xtm4 were not identical.

Unexpectedly the substitution of cysteine 92 resulted in more stable clones. The consequence of substitutions here is that the disulphide bond cysteine 92 to cysteine 111 would be disrupted and disappear. In addition no mutants more than twice as thermostable as the wild-type were found when susbtituting the cysteine at position 111 alone. The above observation can be understood after having sequenced the two clones xtm 9 and xtm10. It turned out that these clones had not in fact had a disrupted disulphide bond. Instead in both cases the mutation S59N was found (SEQ. ID. No. 2). Surprisingly the mutation was outside the original selected mutagenesis regions. However it emphasises again the success by mutating a serine residue. In particular the serine at position 59 is fully exposed at the surface of the molecule.

(Note that both SEQ. ID. Nos. 2 and 4 give the full preprosequnce of the mutant endoxylanases. The signal and preprosequences account for the first 27 amino acids, so the mature sequence starts at Ser²⁸. The numbering for mutation purposes is based on the mature sequence and so to obtain the number in SEQ. ID. Nos. 2 and 4, add 27 (so S93L, 93+27=120, see Ser¹²⁰).

For threonine residues, seven positions were mutated resulting in improved stress resistance. Only 3 of these positions could be unambiguously assigned to a threonine substitution. In 4 cases the position was a part of a mixture of mutants.

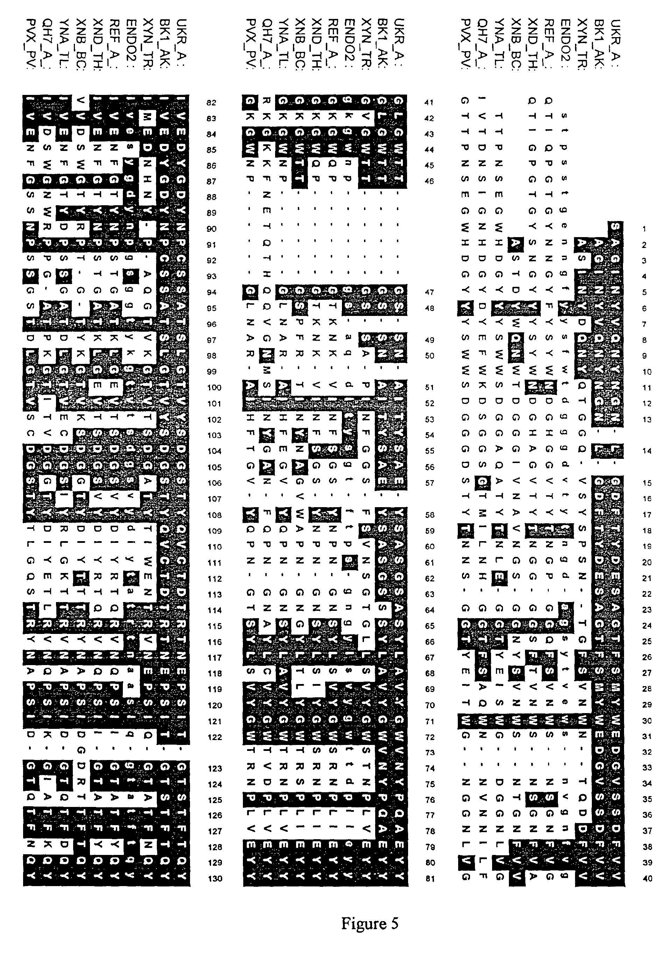

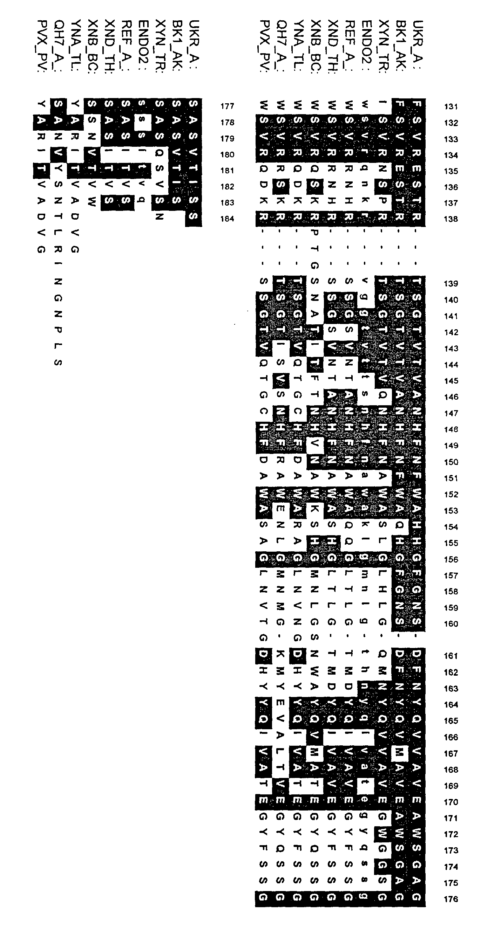

The EndoI xylanase contains three histidine residues in total. The two residues that were substituted both showed improved thermostability. Both histidines, which are neighbours (154, 155) in the sequence as well as in the 3D structure, are completely exposed to the solvent. The two neighbouring histidines is not necessarily an advantage as can be deduced from sequence alignment of Family 11 xylanases (see Figure 5). EndoI is the only species having this feature. Even in the very close homologue (*A.kawachii*, BK1_AK) position 154 is a glutamine. This suggests that the EndoI sequence is not quite optimal here and replacing at least one histidines improves the conformational stability of the molecule.

For many of the clones the xylanase activity was determined (these were given xtm numbers) relative to the wild-type EndoI enzyme, and the results are shown in Table 9.

**Table 9:**

| Activity results for selected clones in shake flask experiments | | | |
|---|---|---|---|
| Xylanase (mutant code no. or wild-type) | Activity in Shake flask EXU/ml | Half life lab-test (min) | MTP well: Clone no. and position no. of mutation |
| XTM1 | 329 | 122 | A:f5 91-93 |
| XTM2 | 276 | 62 | A:g6 91-93 |
| XTM3 | 90 | 60 | A:f10 91-93 |
| XTM4 | 74 | 433 | A:h3 91-93 |
| XTM6 | 35 | 112 | A:f12 92 |
| XTM8 | 47 | 68 | A:a4 92 |
| XTM9 | 92 | 115 | A:g12 92 |
| XTM10 | 68 | 148 | A:g1 92 |
| XTM11 | 99 | 52 | C:h2 115-116 |
| XTM15 | 78 | 65 | C:b8 124 |
| XTM16 | 227 | 70 | C:e7 124 |
| XTM17 | 78 | 53 | C:e11 139-140 |
| XTM18 | 69 | 82 | C:e10 139-140 |
| XTM21 | 33 | 118 | D:b1 136 |
| XTM23 | 86 | 81 | D:f12 136 |
| XTM24 | 153 | 69 | D:b9 136 |
| XTM25 | 182 | 85 | D:a4 136 |
| XTM28 | 176 | 52 | E:g6 154 |
| XTM31 | 39 | 58 | G:c12 137 |
| wt103 | 98 | 42 | |
| wt104 | 28 | 43 | |

### REFERENCES

1. Fushinobu *et al*, Protein Engineering 11(12):1121-1128 (1998)
2. Joshi *et al*, J. Mol. Biol. 299: 255-279 (2000)
3. Arase *et al*, FEBS Letters 316(2): 123-127 (January 1993)
4. US Patent No. 5,405,769 (Campbell/National Research Council of Canada)
5. Wakarchuk *et al*, Protein Engineering 7(11): 1379-1386 (1994)
6. WO-A-94/24270 (Campbell/National Research Council of Canada).
7. Vehmaanpera *et al*, Poster P51, presented at TriCel 97, Ghent, Belgium (1997)
8. EP-A-0,828,002 (Sung/National Research Council of Canada)
9. Gruber *et al*, Biochemistry 37(39): 13475-13485 (1998)
10. Törrönen *et al*, Biochemistry 34: 847-856 (1995)
11. Krengel *et al*, J. Mol Biol. 263: 70-78 (1976)
12. EP-A-0,463,706 (Gist-brocades B.V.)
13. De Graaf *et al*, Molecular Microbiology 12(3): 479-490 (1994)
14. Ford *et al*, Protein Expression and Purification, 2: 95-107 (1991)
15. WO-A-94/14965 (van Ooyen/Gist-brocades)
16. Sambrook *et al*, "Molecular Cloning: a laboratory manual", 2^{nd} Edition, Cold Spring Harbor Laboratories, Cold Spring Harbor, New York (1989)
17. Innis *et al* (1990) "PCR protocols, a guide to methods and applications" Academic Press, San Diego
18. Ito *et al*, J. Bacteriol. 153: 163-168 (1983)
19. Lever, M., Powell, J.C., Killip, M., Small, C.W. (1973) J. Lab. Clin. Med. 82: 649-655
20. Kabsch & Sander, Biopolymers, 22: 2577-2637
21. Cunningham and Wells, Science, 244: 1081-1085 (1989)
22. de Vos *et al*, Science, 255: 306-312 (1992)
23. Smith *et al*, J. Mol. Biol., 224: 899-904 (1992)
24. Wlodaver *et al*, FEBS Lett., 309: 59-64 (1992)
25. WO-A-99/32617
26. van Zeijl, C. et al, J. of Biotechnol. 59: 221-224 (1998)
27. Goosen *et al*, "Transformation and Gene Manipulation in Filamentous Fungi: an overview" in: Handbook of Applied Mycology, Vol. 4 (1992)
28. Romanos *et al*, Yeast 8:423-488 (1992)
29. EP-A-0,635,574
30. WO-A-98/46772
31. Alenkso and Clutterbuck, Fungal Genet. Biol 21: 373-397 (1997)
32. WO-A-98/04726
33. WO-A-98/30707
34. EP-A-0,184,433
35. EP-A-0,284,603
36. EP-A-0,134,048
37. EP-A-0,253,455
38. EP-A-0,096,340
39. EP-A-0,301,670
40. EP-A-0,449,375
41. WO-A-91/14772
42. Sander & Schneider, Proteins, 9:56-68 (1991)
43. WO-A-98/46774 (Gist-brocades)
44. EP-A-0,340,878 (UCI)

## Claims

1. A protein comprising a fungal xylanase modified to increase thermostability, where the modification is at an exposed serine residue or within residues 90 to 160.

2. A protein according to claim 1 wherein the xylanases is modified to have greater thermostability and/or no reduced xylanase activity when compared with the wild-type xylanase, or the xylanase is from *Aspergillus.*

3. A protein according to claim 1 or 2 which has a thermostability that is at least 1.5 times as much as the wild-type endoxylanase I and/or the endoxylanase is from *Aspergillus tubingensis* or *Aspergillus niger.*

4. A protein according to any preceding claim which has a thermostability that is at least twice as much as a wild-type endoxylanase and/or is a modified endo-1,4-β-xylanase I (*xlnA*).

5. A protein according to claim 1 wherein the modification is:
(a) outside the active cleft region;
(b) at a residue located on the outside of the polypeptide; and
(c) at a proline, cysteine, serine, alanine, leucine, valine, tyrosine, asparagine, threonine, arginine, glutamine, histidine and/or aspartic acid residue.

6. A protein according to any preceding claim wherein:
(i) it is a G-type endoxylanase;
(ii) the modification is at a serine, alanine, valine, tyrosine, aspartic acid or threonine residue;
(iii) the protein has one or more additional modifications to the modification(s) to increase thermostability; and/or
(iv) the protein is a fragment of a fungal xylanase where the fragment possesses the modification(s) to increase thermostability.

7. A protein according to any preceding claim wherein the modification is at a serine or threonine residue and/or the protein has an optimum pH of from 2 to 5.5.

8. A protein according to any preceding claim wherein the modification is:
(d) other than the introduction of a new disulphide bridge;
(e) does not result in a significant structural change to the molecule; and/or
(f) does not reduce the activity of the xylanase when compared to the wild-type.

9. A protein according to any preceding claim wherein the modification is:
(g) at one or more of the positions 91-104, 108, 113-117, 122-126, 136-140, 145 or 153-160;
(h) at a residue in the B7, B8 or B9 antiparallel strand;
(i) is a single substituion of one residue for another residue; and/or
(j) is a substitution by a Pro, Gly, Ala, Leu, Val, Ile, Phe, Thr, Cys, Thr, Asn, His, Arg or Asp residue.

10. A protein according to any preceding claim wherein the modification is at the position 91, 92, 93, 94, 95, 98, 103, 108 or 155 and/or at any of the serine residues at positions 22, 27, 48, 49, 55, 59, 61, 173, 177, 179 or 183.

11. A protein according to any preceding claim wherein the modification is at position 136, 103, 98, 93, 122, 95, 94, 92 or 91 and/or the modification is a substitution of the original residue by a Cys, Thr, Asn, His, Arg or Asp residue.

12. A protein according to any of the preceding claims wherein the mutation is S93L or S59N.

13. A polynucleotide encoding a protein according to any preceding claim.

14. A vector comprising a polynucleotide sequence according to claim 13.

15. A vector according to claim 14 which is an expression vector, such as where a DNA sequence according to claim 13 is operably linked to a regulatory sequence.

16. A host cell which comprises, as a heterologous sequence, a polynucleotide according to claim 13.

17. A host cell which expresses heterologously a protein according to any of claims 1 to 12.

18. A host cell transformed with a polynucleotide according to claim 13 or a vector according to claim 14 or15.

19. A process of producing a polypeptide according to any of claims 1 to 12, the process comprising culturing a host cell as defined in any of claims 16 to 18 under conditions that provide for expression of the polypeptide.

20. A composition comprising a polypeptide according to any one of claims 1 to 12.

21. A composition according to claim 20 which further comprises a polypeptide having cellulase, endo-arabinanase, rhamnogalacturonase or polygalacturonase activity.

22. A method of treating a plant or xylan-containing material, the method comprising contacting the material with a protein according to any one of claims 1 to 12 or a composition according to claim 20 or claim 21.

23. A method according to claim 22 wherein the treatment comprises degrading, hydrolysing or modifying xylan in the material or degrading or modifying plant cell walls.

24. A method according to claim 22 or 23 wherein the treatment comprises cleaving of xylopyranosyl subunits and/or the material comprises a plant, plant pulp, plant extract or an edible foodstuff or ingredient therefor.

25. A processed material obtainable by contacting a plant or xylan-containing material with a polypeptide according to any one of claims 1 to 12 or a composition according to claim 20 or 21, or which results from a method according to any of claim 22 to 24.

26. A method according to any of claims 22 to 25 which reduces the viscosity of the material, degrades or hydrolyses xylan contained in the material or improves clarity or filterability of the material.

27. Use of a polypeptide according to any one of claims 1 to 12 or a composition according to claim 20 or claim 21 in a method of treating plant material, improving filterability and/or reducing viscosity of xylan-containing liquids, improving filterability or clarifying alcoholic liquids (e.g. beer, wine) or fruit or vegetable juices, hydrolysing agricultural residues, in recycling materials (e.g. containing paper) in paper making for thickening foodstuffs and/or extracting desirable materials (e.g. coffee, plant oil, starch), processing plant pulp, juice or extract, which method optionally comprises incubating the pulp, juice or extract with the polypeptide or composition to at least partially degrade xylan.

28. An (animal) feed, food or foodstuff comprising a polypeptide according to any one of claims 1 to 12.

29. The use of a polypeptide according to any of claims 1 to 12 in brewing, beer or wine-making, distilling, recycling, biomethanation, dental hygiene, leather treatment, paper manufacture, fruit or vegetable juice or extract treatment, textile treatment or manufacture, baking or bread making, treating flower bulbs, preparation of food or foodstuffs or in an animal feed.

30. A food or foodstuff according to claim 28 which is an alcoholic beverage, bread, dough or tea.

31. A transgenic organism such as a plant or part thereof, comprising a cell according any of claims 16 to 18.
